# EUROPEAN PATENT APPLICATION

(11) **EP 4 454 658 A1**
(43) Date of publication of application: **30.10.2024**
(21) Application number: 22912004.3
(22) Date of filing: 22.12.2022
(51) Int. Cl.: A61K 38/16, A61K 47/68, A61K 47/60, A61P 1/16

(54) **LIVER-TARGETED SUBSTANCE AND USE THEREOF**

(30) Priority: 22.12.2021 KR 20210185350
(71) Applicant: Hanmi Pharm. Co., Ltd., Hwaseong-si, Gyeonggi-do 18536 (KR)
(72) Inventor: KIM, Jung Kuk, Hwaseong-si Gyeonggi-do 18469 (KR); CHOI, Jae Hyuk, Hwaseong-si Gyeonggi-do 18469 (KR); OH, Euh Lim, Hwaseong-si Gyeonggi-do 18469 (KR); LEE, A Ram, Hwaseong-si Gyeonggi-do 18469 (KR); KIM, Sang Yun, Hwaseong-si Gyeonggi-do 18469 (KR)
(74) Representative: Regimbeau
(86) International application number: PCT/KR2022/021130
(87) International publication number: WO 2023/121371

(57) **Abstract**

The present invention relates to a liver-targeted drug, and therapeutic use thereof for diseases requiring drug action in the liver. Additionally, the present invention relates to a method in which a substance having activity on glucagon is used so as to induce targeting of the drug to the liver tissue or an increase in distribution in the liver tissue after *in vivo* administration.

## Description

### [Technical Field]

The present invention relates to a liver-targeted drug and therapeutic use thereof for diseases requiring drug action in the liver. Additionally, the present invention relates to a method in which a liver-targeted drug is used so as to induce targeting of the drug to the liver tissue or an increase in distribution in the liver tissue after *in vivo* administration.

### [Background Art]

The liver is one of the largest organs in our body and plays an important role in various and comprehensive metabolic processes. Among the many vegetable or animal substances that we ingest and the metabolites produced by the performance of biological functions, there are some substances that are beneficial to the body, but there are also many substances that are harmful. The liver performs chemical processes that help the beneficial substances among these substances to be properly utilized by the body, and helps harmful substances to be safely excreted out of the body through urine or feces by chemical metabolic processes. The liver plays an essential role in biological functions by synthesizing and secreting various types of proteins, fats, and carbohydrates necessary for our body. Therefore, when the liver function deteriorates, various functional abnormalities may appear. Insufficient production of coagulation factors involved in blood clotting leads to bleeding, and frequent bleeding occurs from weak gums, *etc.* Additionally, in patients with liver cirrhosis, insulin is not properly degraded, and glycogen storage in the liver is insufficient, leading to hypoglycemia due to fasting. The liver also plays a pivotal role in defense against bacterial invasion. In particular, among the cells in the liver, Kupffer cells mainly perform a phagocytic action by engulfing foreign substances or bacteria, and also play a role in inducing natural immune action in the body by exposing viruses that enter the body to the immune system. Additionally, bile, the major substance synthesized and secreted by liver cells, is formed in approximately 800 to 1000 cc per day, and its main components are water, electrolytes, bile acids, cholesterol, phospholipids, and bilirubin. The main function of bile is that bile acids in bile play an important role in digesting and absorbing fat and fat-soluble vitamins in the small intestine, and bile itself plays a role in excreting a large amount of waste products produced in the body through feces. Diseases that require drug action in the liver developed by various causes include various diseases, e.g., as liver disease, hepatitis caused by viral infection, primary biliary cirrhosis, non-alcoholic steatohepatitis disease, liver cirrhosis, liver cancer, *etc.*

Primary biliary cirrhosis (PBC) is a rare disease, and is a chronic inflammatory disease with biliary fibrous strictures and chronic cholestasis. It causes fibrous strictures in part or all of the extrahepatic and intrahepatic biliary tracts, resulting in chronic cholestasis and biliary cirrhosis, and eventually leading to liver failure due to damaged liver cells. It occurs most often in middle-aged women, and 90 to 95% of patients have a positive result for antimitochondrial antibody, while showing cholestatic abnormalities, such as elevated alkaline phosphatase (ALP), gamma glutamyl transpeptidase (GGT), and bilirubin, in biochemical tests. There is currently no specific treatment drug for primary biliary cirrhosis, and for cholestatic diseases, ursodeoxycholic acid (UDCA), which plays a role in protecting and recovering liver cells by improving cholestasis, has been used, and obeticholic acid from Intercept Pharmaceuticals, Inc., a drug that improves fatty liver disease, was recently approved as an indication in the United States.

In particular, non-alcoholic fatty liver disease (NAFLD) is a type of a disease, showing histological organization similar to those of alcoholic liver disease, although is not associated with alcohol consumption, and is a disease encompassing non-alcoholic fatty liver (NAFL), non-alcoholic steatohepatitis (NASH), liver cirrhosis, and hepatocellular carcinomas. The occurrence of non-alcoholic fatty liver diseases increases with an increase in population with obesity and diabetes. In Korea, the annual incidence has reached approximately 16%.

The non-alcoholic fatty liver disease is known to be caused by various etiologies such as insulin resistance, lipotoxicity, inflammatory responses, *etc.* Among them, the most common etiology is insulin resistance.

Extensive effort has been made to improve insulin resistance to prevent/treat the non-alcoholic fatty liver disease. For example, currently, clinical trials for thiazolidnedinones (TZD) or metformin, a kind of insulin sensitizer, have been actively conducted (Hepatology (2003) 38: 1008-17, J Clin Invest (2001) 108: 1167-74).

Meanwhile, in order to treat the liver diseases described above, it is one of the important technical challenges to allow drugs to reach the liver at high concentrations to be distributed. (Korean Patent Publication No. KR 10-2013-0131227).

Liver-targeted drugs can optimize drug treatment by minimizing the side effects of existing drugs and maximizing their efficacy and effectiveness, enabling efficient delivery of the required amount of drug. This increases the amount of drug reaching the target area, and thus increasing bioavailability, and enables more effective treatment, reduces side effects by preventing delivery to areas other than the target area, and improves the patient's response to the drug, thereby improving patient compliance.

### [Disclosure]

### [Technical Problem]

In order to increase the therapeutic effect of diseases requiring drug action in the liver, it is required to develop an excellent liver-targeted drug that has a therapeutic effect and has a high degree of distribution of the drug in the liver among the body organs of an administered subject.

### [Technical Solution]

It is one object of the present invention to provide a pharmaceutical composition including a liver-targeted drug, specifically a pharmaceutical composition, which has a high distribution of the drug in the liver among the body organs of an administered subject including the liver-targeted drug.

It is another object of the present invention to provide a pharmaceutical composition including a liver-targeted drug, specifically a pharmaceutical composition for prevention or treatment of diseases requiring drug action in the liver.

It is still another object of the present invention to provide a method for preventing or treating diseases requiring drug action in the liver, including: administering the pharmaceutical composition or a physiologically active substance targeted to the liver tissue to a subject in need thereof.

It is yet another object of the present invention to provide the use of the liver-targeted drug, specifically a physiologically active substance targeted to the liver tissue, for use in the preparation of a drug for preventing or treating diseases requiring drug action in the liver.

It is even another object of the present invention to provide a method for inducing targeting to the liver by administering the liver-targeted drug, specifically a physiologically active substance targeted to the liver tissue, to a subject in need thereof.

It is further another object of the present invention to provide a method in which the liver-targeted drug, specifically a physiologically active substance targeted to the liver tissue, is administered to a subject in need thereof, thereby inducing an increase in distribution of the physiologically active substance in the liver tissue.

### [Advantageous Effects]

The drug of the present invention (*e.g.*, a physiologically active protein or peptide derivative) containing a substance that binds to a hepatocyte receptor reaches the liver tissue more effectively from the blood vessels and thus having a binding affinity to a physiologically active substance, which is relatively highly distributed among the body organs after *in vivo* administration, specifically a glucagon receptor. Accordingly, it is not only provided as a physiologically substance that is relatively highly distributed in the liver tissue, but also has a high degree of distribution in the liver within about 7 days, and thus can be applied to the treatment of diseases requiring drug action in the liver, such as various liver diseases, *etc.,* while reducing the patient's inconvenience in taking medication.

### [Detailed Description of the Invention]

One aspect for implementing the present invention provides a pharmaceutical composition including a liver-targeted drug, specifically a pharmaceutical composition, which has a high distribution of the drug in the liver among the body organs of an administered subject.

In one embodiment, the liver-targeted drug is a physiologically active substance targeted to the liver tissue, and is in the form of a peptide including the amino acid sequence of General Formula 1 below or a long-acting conjugate including the same.

In one embodiment, the liver-targeted drug is a peptide including the amino acid sequence of General Formula 1 below: 
Xaa1-Xaa2-Xaa3-Gly-Thr-Phe-Xaa7-Ser-Asp-Xaa10-Ser-Xaa12-Xaa13-Xaa14-
Xaa15-Xaa16-Xaa17-Xaa18-Xaa19-Xaa20-Xaa21-Phe-Xaa23-Xaa24-Trp-Leu-Xaa27-X
aa28-Xaa29-Xaa30-R1 (General Formula 1, SEQ ID NO: 103)
 wherein in the General Formula 1 above,
Xaa1 is histidine (His, H), 4-imidazoacetyl (CA), or tyrosine (Tyr, Y);
Xaa2 is glycine (Gly, G), α-methyl-glutamic acid, or aminoisobutyric acid (Aib);
Xaa3 is glutamic acid (Glu, E) or glutamine (Gln, Q);
Xaa7 is threonine (Thr, T) or isoleucine (Ile, I);
Xaa10 is leucine (Leu, L), tyrosine (Tyr, Y), lysine (Lys, K), cysteine (Cys, C), or valine (Val, V);
Xaa12 is lysine (Lys, K), serine (Ser, S), or isoleucine (Ile, I);
Xaa13 is glutamine (Gln, Q), tyrosine (Tyr, Y), alanine (Ala, A), or cysteine (Cys, C);
Xaa14 is leucine (Leu, L), methionine (Met, M), or tyrosine (Tyr, Y);
Xaa15 is cysteine (Cys, C), aspartic acid (Asp, D), glutamic acid (Glu, E), or leucine (Leu, L);
Xaa16 is glycine (Gly, G), glutamic acid (Glu, E), or serine (Ser, S);
Xaa17 is glutamine (Gln, Q), arginine (Arg, R), isoleucine (Ile, I), glutamic acid (Glu, E), cysteine (Cys, C), or lysine (Lys, K);
Xaa18 is alanine (Ala, A), glutamine (Gln, Q), arginine (Arg, R), or histidine (His, H);
Xaa19 is alanine (Ala, A), glutamine (Gln, Q), cysteine (Cys, C), or valine (Val, V);
Xaa20 is lysine (Lys, K), glutamine (Gln, Q), or arginine (Arg, R);
Xaa21 is glutamic acid (Glu, E), glutamine (Gln, Q), leucine (Leu, L), cysteine (Cys, C), or aspartic acid (Asp, D);
Xaa23 is isoleucine (Ile, I) or valine (Val, V);
Xaa24 is alanine (Ala, A), glutamine (Gln, Q), cysteine (Cys, C), asparagine (Asn, N), aspartic acid (Asp, D), or glutamic acid (Glu, E);
Xaa27 is valine (Val, V), leucine (Leu, L), lysine (Lys, K) or methionine (Met, M);
Xaa28 is cysteine (Cys, C), lysine (Lys, K), alanine (Ala, A), asparagine (Asn, N), or aspartic acid (Asp, D);
Xaa29 is cysteine (Cys, C), glycine (Gly, G), glutamine (Gln, Q), threonine (Thr, T), glutamic acid (Glu, E), or histidine (His, H);
Xaa30 is cysteine (Cys, C), glycine (Gly, G), lysine (Lys, K), or histidine (His, H), or is absent; and
R1 is cysteine (Cys, C), GKKNDWKHNIT (SEQ ID NO: 106), m-SSGAPPPS-n (SEQ ID NO: 107), or m-SSGQPPPS-n (SEQ ID NO: 108), or is absent;
   wherein,
   m is Cys, Pro, or Gly-Pro;
   n is Cys, Gly, Ser, or His-Gly, or is absent.

In another embodiment, the peptide is in the form of a long-acting conjugate, and the long-acting conjugate is represented by Formula 1 below:

[Formula 1] X-L-F

wherein,
X is a peptide comprising the amino acid sequence of General Formula 1 above;
L is a linker containing an ethylene glycol repeating unit;
F is an immunoglobulin Fc region; and
- represents a covalent bond between X and L, and between L and F.

As the pharmaceutical composition according to any one of the above-described embodiments, the body organ is the liver, heart, lung, large intestine, spleen, pancreas, adipose tissue, small intestine, stomach, muscle, kidney and brain, and the composition is the most highly distributed in the liver among each body organ.

As the pharmaceutical composition according to any one of the above-described embodiments, the composition is for use in the prevention or treatment of diseases requiring drug action in the liver.

As the pharmaceutical composition according to any one of the above-described embodiments, the liver-targeted drug has a tissue-to-serum ratio (T/S ratio) in the liver after administration of at least one selected from the following:
(a) a T/S ratio of 25% to 50% at 2 to 5 hours after administration;
(b) a T/S ratio of 40% to 60% at 40 to 50 hours after administration; and
(c) a T/S ratio of 45% to 75% at 160 to 180 hours after administration.

As the pharmaceutical composition according to any one of the above-described embodiments, the liver-targeted drug has a tissue-to-serum ratio (T/S ratio) in the liver after administration of at least one selected from the following:
(a) a T/S ratio of about 30 to about 50% at about 2 to about 5 hours after administration;
(b) a T/S ratio of about 45% to about 55% at about 40 to about 50 hours after administration; and
(c) a T/S ratio of about 50% to about 65% at about 160 to about 180 hours after administration.

As the pharmaceutical composition according to any one of the above-described embodiments, the liver-targeted drug has a T/S ratio in the liver after administration of at least one selected from the following:
(a) a T/S ratio of 35% to 45% at 4 hours after administration;
(b) a T/S ratio of 45% to 55% at 2 days after administration; and
(c) a T/S ratio of 55% to 70% at 7 days after administration.

As the pharmaceutical composition according to any one of the above-described embodiments, the liver-targeted drug has a T/S ratio in the liver after administration of at least one selected from the following:
(a) a T/S ratio of about 41 % to about 43% at about 4 hours after administration;
(b) a T/S ratio of about 48% to about 52% at about 2 days after administration; and
(c) a T/S ratio of about 60% to about 65% at about 7 days after administration.

As the pharmaceutical composition according to any one of the above-described embodiments, the liver-targeted drug has a distribution ratio in the liver relative to lung tissue after administration of 1: about 2 to about 4.

As the pharmaceutical composition according to any one of the above-described embodiments, the liver-targeted drug has a distribution ratio in the liver relative to lung tissue after administration of 1: about 3 to about 4.

As the pharmaceutical composition according to any one of the above-described embodiments, the liver-targeted drug has a distribution ratio in the liver relative to lung tissue after administration of 1: about 3.5 to about 4.

As the pharmaceutical composition according to any one of the above-described embodiments, the distribution ratio in the liver relative to lung tissue after administration is the distribution ratio at about 40 to about 180 hours after administration.

As the pharmaceutical composition according to any one of the above-described embodiments, the distribution ratio in the liver relative to lung tissue after administration is the distribution ratio at about 2 to about 7 days after administration.

As the pharmaceutical composition according to any one of the above-described embodiments, the liver-targeted drug has one of the following:
(a) a distribution ratio in the liver relative to the heart of 1:1.5 to 3.0 at 2 to 5 hours after administration;
(b) a distribution ratio in the liver relative to the heart of 1:2.0 to 3.0 at 40 to 50 hours after administration; and
(c) a distribution ratio in the liver relative to the heart at 160 to 180 hours after administration of 1: 5.5 to 7.0.

As the pharmaceutical composition according to any one of the above-described embodiments, the liver-targeted drug is in the form of a peptide which is relatively highly distributed in the liver among the body organs after administration, or a long-acting conjugate containing the same.

As the pharmaceutical composition according to any one of the above-described embodiments, the liver-targeted drug has therapeutic activity for diseases requiring drug action in the liver.

As the pharmaceutical composition according to any one of the above-described embodiments, the disease requiring drug action in the liver is a liver disease.

As the pharmaceutical composition according to any one of the above-described embodiments, the liver disease is liver cancer.

As the pharmaceutical composition according to any one of the above-described embodiments, the liver-targeted drug is a protein or peptide including a peptide sequence having a binding affinity to a glucagon receptor.

As the pharmaceutical composition according to any one of the above-described embodiments,
wherein in the General Formula 1 above,
Xaa2 is glycine, α-methyl-glutamic acid, or Aib;
Xaa7 is threonine;
Xaa10 is tyrosine, cysteine, or valine;
Xaa12 is lysine or isoleucine;
Xaa13 is tyrosine, alanine, glutamine, or cysteine;
Xaa14 is leucine, tyrosine, or methionine;
Xaa15 is cysteine, leucine, glutamic acid, or aspartic acid;
Xaa17 is glutamine, arginine, isoleucine, cysteine, glutamic acid, or lysine;
Xaa18 is alanine, glutamine, arginine, or histidine;
Xaa19 is alanine, glutamine, valine, or cysteine;
Xaa20 is lysine, arginine, or glutamine;
Xaa21 is glutamic acid, glutamine, leucine, cysteine, or aspartic acid;
Xaa23 is isoleucine or valine;
Xaa24 is cysteine, alanine, glutamine, asparagine, glutamic acid, or aspartic acid; and
Xaa27 is leucine or lysine.

As the pharmaceutical composition according to any one of the above-described embodiments, the peptide includes the amino acid sequence represented by General Formula 2 below: 
Xaa1-Xaa2-Gln-Gly-Thr-Phe-Thr-Ser-Asp-Xaa10-Ser-Lys-Xaa13-Xaa14-Xaa15-
Xaa16-Xaa17-Xaa18-Xaa19-Xaa20-Xaa21-Phe-Xaa23-Xaa24-Trp-Leu-Leu-Xaa28-Xaa
29-Xaa30-Xaa31-Ser-Ser-Gly-Gln-Pro-Pro-Pro-Ser-Xaa40 (General Formula 2, SEQ ID
NO: 104)
 wherein in the General Formula 2 above,
Xaa1 is 4-imidazoacetyl, histidine, or tyrosine;
Xaa2 is glycine, α-methyl-glutamic acid, or Aib;
Xaa10 is tyrosine, or cysteine;
Xaa13 is alanine, glutamine, tyrosine, or cysteine;
Xaa14 is leucine, methionine, or tyrosine;
Xaa15 is aspartic acid, glutamic acid, or leucine;
Xaa16 is glycine, glutamic acid, or serine;
Xaa17 is glutamine, arginine, isoleucine, glutamic acid, cysteine, or lysine;
Xaa18 is alanine, glutamine, arginine, or histidine;
Xaa19 is alanine, glutamine, cysteine, or valine;
Xaa20 is lysine, glutamine, or arginine;
Xaa21 is cysteine, glutamic acid, glutamine, leucine, or aspartic acid;
Xaa23 is isoleucine or valine;
Xaa24 is cysteine, alanine, glutamine, asparagine, or glutamic acid;
Xaa28 is lysine, cysteine, asparagine, or aspartic acid;
Xaa29 is glycine, glutamine, cysteine, or histidine;
Xaa30 is cysteine, glycine, lysine, or histidine;
Xaa31 is proline or cysteine; and
Xaa40 is cysteine, or is absent.

As the pharmaceutical composition according to any one of the above-described embodiments,
in the General Formula 1 above,
Xaa2 is glycine, α-methyl-glutamic acid, or Aib;
Xaa7 is threonine;
Xaa10 is tyrosine, cysteine, or valine;
Xaa12 is lysine or isoleucine;
Xaa13 is tyrosine, alanine, or cysteine;
Xaa14 is leucine or methionine;
Xaa15 is cysteine or aspartic acid;
Xaa17 is glutamine, arginine, isoleucine, cysteine, or lysine;
Xaa18 is alanine, arginine, or histidine;
Xaa19 is alanine, glutamine, or cysteine;
Xaa20 is lysine or glutamine;
Xaa21 is glutamic acid, cysteine, or aspartic acid;
Xaa23 is valine;
Xaa24 is alanine, glutamine, cysteine, asparagine, or aspartic acid; and
Xaa27 is leucine or lysine.

As the pharmaceutical composition according to any one of the above-described embodiments,
in the General Formula 2 above,
Xaa13 is alanine, tyrosine, or cysteine;
Xaa15 is aspartic acid or glutamic acid;
Xaa17 is glutamine, arginine, cysteine, or lysine;
Xaa18 is alanine, arginine, or histidine;
Xaa21 is cysteine, glutamic acid, glutamine, or aspartic acid;
Xaa23 is isoleucine or valine;
Xaa24 is cysteine, glutamine, or asparagine;
Xaa28 is cysteine, asparagine, or aspartic acid;
Xaa29 is glutamine, cysteine, or histidine; and
Xaa30 is cysteine, lysine, or histidine.

As the pharmaceutical composition according to any one of the above-described embodiments,
in the General Formula 1 above,
Xaa2 is α-methyl-glutamic acid or Aib;
Xaa7 is threonine;
Xaa10 is tyrosine or cysteine;
Xaa12 is lysine or isoleucine;
Xaa13 is tyrosine, alanine, or cysteine;
Xaa14 is leucine or methionine;
Xaa15 is cysteine or aspartic acid;
Xaa16 is glutamic acid;
Xaa17 is arginine, isoleucine, cysteine, or lysine;
Xaa18 is alanine, arginine, or histidine;
Xaa19 is alanine, glutamine, or cysteine;
Xaa20 is lysine or glutamine;
Xaa21 is glutamic acid or aspartic acid;
Xaa23 is valine;
Xaa24 is glutamine, asparagine, or aspartic acid;
Xaa27 is leucine; and
Xaa28 is cysteine, alanine, asparagine, or aspartic acid.

As the pharmaceutical composition according to any one of the above-described embodiments,
in the General Formula 1 above,
Xaa1 is histidine or 4-imidazoacetyl;
Xaa2 is α-methyl-glutamic acid or Aib;
Xaa3 is glutamine;
Xaa7 is threonine;
Xaa10 is tyrosine;
Xaa12 is isoleucine,
Xaa13 is alanine or cysteine;
Xaa14 is methionine,
Xaa15 is aspartic acid;
Xaa16 is glutamic acid;
Xaa17 is isoleucine or lysine;
Xaa18 is alanine or histidine;
Xaa19 is glutamine or cysteine;
Xaa20 is lysine;
Xaa21 is aspartic acid;
Xaa23 is valine,
Xaa24 is asparagine;
Xaa27 is leucine;
Xaa28 is alanine or asparagine;
Xaa29 is glutamine or threonine; and
Xaa30 is cysteine or lysine, or is absent.

As the pharmaceutical composition according to any one of the above-described embodiments, the peptide is a peptide including the amino acid sequence of General Formula 3 below: 
Xaa1-Xaa2-Gln-Gly-Thr-Phe-Thr-Ser-Asp-Tyr-Ser-Lys-Xaa13-Leu-Asp-Glu-Xaa
17-Xaa18-Xaa19-Lys-Xaa21-Phe-Val-Xaa24-Trp-Leu-Leu-Xaa28-Xaa29-Xaa30-Xaa31-
Ser-Ser-Gly-Gln-Pro-Pro-Pro-Ser-Xaa40 (General Formula 3, SEQ ID NO: 105)
 wherein in the General Formula 3 above,
Xaa1 is histidine or tyrosine;
Xaa2 is α-methyl-glutamic acid or Aib;
Xaa13 is alanine, tyrosine or cysteine;
Xaa17 is arginine, cysteine, or lysine;
Xaa18 is alanine or arginine;
Xaa19 is alanine or cysteine;
Xaa21 is glutamic acid or aspartic acid;
Xaa24 is glutamine or asparagine;
Xaa28 is cysteine or aspartic acid;
Xaa29 is cysteine, histidine, or glutamine;
Xaa30 is cysteine or histidine;
Xaa31 is proline or cysteine; and
Xaa40 is cysteine, or is absent.

As the pharmaceutical composition according to any one of the above-described embodiments,
R1 is cysteine, GKKNDWKHNIT (SEQ ID NO: 106), CSSGQPPPS (SEQ ID NO: 109), GPSSGAPPPS (SEQ ID NO: 110), GPSSGAPPPSC (SEQ ID NO: 111), PSSGAPPPS (SEQ ID NO: 112), PSSGAPPPSG (SEQ ID NO: 113), PSSGAPPPSHG (SEQ ID NO: 114), PSSGAPPPSS (SEQ ID NO: 115), PSSGQPPPS (SEQ ID NO: 116), or PSSGQPPPSC (SEQ ID NO: 117), or is absent.

As the pharmaceutical composition according to any one of the above-described embodiments, the peptide includes an amino acid sequence selected from the group consisting of SEQ ID NOS: 1 to 102.

As the pharmaceutical composition according to any one of the above-described embodiments, the Xaa16 amino acid and the Xaa20 amino acid form a ring with each other in the peptide.

As the pharmaceutical composition according to any one of the above-described embodiments, the peptide is amidated at its C-terminus or has a free carboxyl group (-COOH).

As the pharmaceutical composition according to any one of the above-described embodiments, the peptide is amidated at its C-terminus.

As the pharmaceutical composition according to any one of the above-described embodiments, the formula weight of the ethylene glycol repeating unit in L is in the range of 1 to 100 kDa.

As the pharmaceutical composition according to any one of the above-described embodiments, the structure of the Formula 1 above is represented by the structure of Formula 3 below. 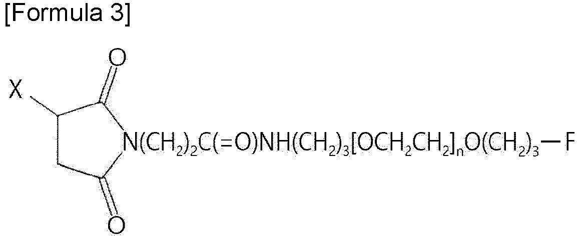 wherein, X and F are as defined in Formula 1 above.

As the pharmaceutical composition according to any one of the above-described embodiments, the ethylene glycol repeating unit has a formula of [OCH₂CH₂]n, wherein n is a natural number, and the average molecular weight of the [OCH₂CH₂]n moiety in the peptide conjugate, for example, the number average molecular weight is determined to be 1 to 100 kDa.

As the pharmaceutical composition according to any one of the above-described embodiments, the value of n is determined such that the average molecular weight of the [OCH₂CH₂]n moiety in the peptide conjugate, for example, the number average molecular weight, is 10 kDa.

As the pharmaceutical composition according to any one of the above-described embodiments, the L is polyethylene glycol.

As the pharmaceutical composition according to any one of the above-described embodiments, the X is linked via a sulfur atom of cysteine in the peptide.

As the pharmaceutical composition according to any one of the above-described embodiments, the F is an IgG Fc region.

As the pharmaceutical composition according to any one of the above-described embodiments, the immunoglobulin Fc fragment is derived from IgG4.

As the pharmaceutical composition according to any one of the above-described embodiments, the F has a structure in which two polypeptide chains are linked by an inter-disulfide bond, and the F is linked only through a nitrogen atom in one of the two chains.

As the pharmaceutical composition according to any one of the above-described embodiments, the F includes a monomer of the amino acid sequence of SEQ ID NO: 139.

As the pharmaceutical composition according to any one of the above-described embodiments, the F is a homodimer of the monomers of the amino acid sequence of SEQ ID NO: 139.

As the pharmaceutical composition according to any one of the above-described embodiments, the F is a homodimer including the amino acid sequence of SEQ ID NO: 140.

As the pharmaceutical composition according to any one of the above-described embodiments, the F is linked through a nitrogen atom of proline at the N-terminus thereof.

As the pharmaceutical composition according to any one of the above-described embodiments, the F, which is an immunoglobulin Fc region, and X are non-glycosylated.

Another aspect for implementing the present invention provides a physiologically active substance targeted to the liver tissue, including a material having a binding affinity to a receptor present in the liver.

Still another aspect for implementing the present invention provides a method for preventing or treating diseases requiring drug action in the liver, including administering the pharmaceutical composition or a physiologically active substance targeted to the liver tissue to a subject in need thereof.

As the pharmaceutical composition according to any one of the above-described embodiments, the disease requiring drug action in the liver is a liver disease.

As the pharmaceutical composition according to any one of the above-described embodiments, the liver disease is a metabolic liver disease.

As the pharmaceutical composition according to any one of the above-described embodiments, the liver disease is at least one disease selected from the group consisting of simple steatosis, non-alcoholic fatty liver, liver inflammation, non-alcoholic steatohepatitis, cholestatic liver disease, liver fibrosis, cirrhosis, liver failure, and liver cancer.

As the pharmaceutical composition according to any one of the above-described embodiments, the cholestatic liver disease is at least one disease selected from the group consisting of primary biliary cirrhosis, primary sclerosing cholangitis, and a combination thereof.

Yet another aspect for implementing the present invention provides the use of the liver-targeted drug, specifically a physiologically active substance targeted to the liver tissue, for use in the preparation of a drug for preventing or treating diseases requiring drug action in the liver.

Even another aspect for implementing the present invention provides a method for inducing targeting to the liver by administering the liver-targeted drug, specifically a physiologically active substance targeted to the liver tissue, to a subject in need thereof.

Further another aspect for implementing the present invention provides a method in which the liver-targeted drug, specifically, a physiologically active substance targeted to the liver tissue, is administered to a subject in need thereof, thereby inducing an increase in distribution of the physiologically active substance in the liver tissue.

### [Mode for Carrying Out the Invention]

Hereinafter, the present invention will be described in more detail.

Meanwhile, each of the explanations and exemplary embodiments disclosed herein can be applied to each other explanation and exemplary embodiment. That is, all of the combinations of various factors disclosed herein belong to the scope of the present invention. Moreover, the scope of the present invention should not be limited by the specific disclosure provided hereinbelow.

Additionally, those of ordinary skill in the art may be able to recognize or confirm, using only conventional experimentation, many equivalents to the particular aspects of the disclosure described herein. Furthermore, it is also intended that these equivalents be included in the present invention. Documents disclosed in the present specification may be incorporated herein by reference in their entirety. In addition, a number of papers and patent documents have been cited throughout the present specification, and their citations are indicated. The content of the cited papers and patent documents is incorporated herein by reference in their entirety and the level of technical field to which the present invention belongs and the contents of the present invention will be described more clearly.

Throughout the entire specification of the present invention, not only the conventional one-letter and three-letter codes for naturally occurring amino acids, but also those three-letter codes generally allowed for other amino acids, such as α-aminoisobutyric acid (Aib), Sar (*N*-methylglycine), α-methyl-glutamic acid, *etc.* are used. Additionally, the amino acids mentioned herein are abbreviated according to the nomenclature rules of IUPAC-IUB as follows:

| | |
|---|---|
| alanine Ala, A | arginine Arg, R |
| asparagine Asn, N | aspartic acid Asp, D |
| cysteine Cys, C | glutamic acid Glu, E |
| glutamine, Gln, Q | glycine Gly, G |
| histidine His, H | isoleucine Ile, I |
| leucine Leu, L | lysine Lys, K |
| methionine Met, M | phenylalanine Phe, F |
| proline Pro, P | serine Ser, S |
| threonine Thr, T | tryptophan Trp, W |
| tyrosine Tyr, Y | valine Val, V |

In the present specification, the "Aib" may be used interchangeably with "2-aminoisobutyric acid" or "aminoisobutyric acid", and 2-aminoisobutyric acid and aminoisobutyric acid may be used interchangeably with each other.

One aspect for implementing the present invention provides a composition including a liver-targeted drug, *e.g.,* a pharmaceutical composition. Specifically, one aspect for implementing the present invention relates to a pharmaceutical composition including a liver-targeted drug, which has a high distribution of the drug in the liver among the body organs of an administered subject.

The high distribution in the liver among the body organs may mean that after administration of a liver-targeted drug into the body, the distribution of the drug is highest in the liver, among the body organs consisting of the liver, heart, lung, large intestine, spleen, pancreas, adipose tissue, small intestine, stomach, muscle, kidney and brain, but is not limited thereto.

The pharmaceutical composition may be a pharmaceutical composition for use in the prevention or treatment of diseases requiring drug action in the liver.

As used herein, the term "liver-targeted drug" may refer to a drug that can be targeted to the liver tissue. Targeting to the liver tissue may mean that the distribution of the drug in the liver is higher than that in the other organs after administration, but is not limited thereto. The liver-targeted drug may have therapeutic activity for diseases requiring drug action in the liver.

The liver-targeted drug may have a tissue-to-serum ratio (T/S ratio) in the liver after administration of at least one selected from the following, but is not limited thereto:
(a) a T/S ratio of about 25% to about 50%, about 30% to about 50%, about 35% to about 50%, about 40% to about 45%, about 41% to about 44%, or about 42% to about 43% at about 2 to about 5 hours after administration;
(b) a T/S ratio of about 40% to about 60%, about 45% to about 55%, about 46% to about 54%, about 47% to about 53%, about 48% to about 53%, about 48% to about 53%, about 49% to about 52%, or about 49% to about 51% at about 40 to about 50 hours after administration; and
(c) a T/S ratio of about 45% to about 75%, about 50% to about 65%, about 55% to about 65%, about 58% to about 65%, about 59% to about 65%, about 60% to about 65%, about 61 % to about 65%, about 62% to about 64%, or about 63% to about 64% at about 160 to about 180 hours after administration. The feature may be one or more, two or more, or all three selected from (a), (b) or (c) above, but is not limited thereto.

The liver-targeted drug may have a tissue-to-serum ratio (T/S ratio) in the liver after administration of at least one selected from the following, but is not limited thereto:
(a) a T/S ratio of about 35% to about 45%, about 40% to about 45%, about 41 % to about 43%, about 42% to about 43%, or about 42.4% at about 4 hours after the administration;
(b) a T/S ratio of about 45% to about 55%, about 48% to about 52%, about 49% to about 52%, about 49% to about 51%, about 50% to about 51%, or about 50.1% at about 2 days after the administration; and
(c) a T/S ratio of about 55% to about 70%, about 60% to about 65%, about 61% to about 65%, about 62% to about 64%, about 63% to about 64%, or about 63.7% at about 7 days after the administration. The feature may be one or more, two or more, or all three selected from (a), (b) or (c) above, but is not limited thereto.

The tissue-to-serum ratio (T/S ratio) is the concentration ratio of tissue to serum, which is converted into %, and can be measured by a known method. For example, the tissue-to-serum ratio (T/S ratio) or T/S ratio (%) is calculated by the tissue concentration/serum concentration x 100. In order to measure the concentration, the concentration of a substance is measured by an ELISA method, *etc.,* after extracting the organ.

The higher T/S ratio means that the distribution of an administered substance in the tissue is higher than in the tissue having a low T/S ratio. In general, when a drug is administered into the body, the organs in which the drug is most highly distributed are the lungs and the heart, thus, when the drug has a higher degree of distribution in the liver tissue compared to that in the lung tissue or the heart tissue, this may mean that the liver-targeted drug of the present invention is effectively targeted to the liver tissue. Additionally, tissues that can be compared to the T/S ratio of the liver tissue by measuring the T/S ratio, which can confirm that the liver-targeted drug is effectively targeted to the liver tissue, may be included without limitation.

The liver-targeted drug has a distribution ratio in the liver relative to the lung tissue after administration may be 1: about 2 to about 4.2, 1: about 3 to about 4.2, 1: about 3 to about 4, 1: about 3.2 to about 4, 1: about 3.3 to about 4, 1: about 3.4 to about 4, 1: about 3.5 to about 4, 1: about 3.5 to about 3.9, 1: about 3.5 to about 3.8, 1: about 3.6 to about 3.8, 1: about 3.6 to about 3.9, or 1: about 3.65 to about 3.89, but the distribution ratio is not limited thereto.

The distribution ratio may be measured based on T/S (%), and when the T/S (%) of the lung is set as 1, the multiple of T/S (%) in the liver may be confirmed.

The distribution ratio in the liver relative to the lung tissue after administration may be about 40 hours to about 180 hours, about 45 hours to about 170 hours, or about 2 days to about 7 days after administration, but is not limited thereto.

The distribution ratio in the liver relative to the lung tissue after administration may be 1: about 3.2 to about 3.9, or 1: about 3.6 to about 3.7 at about 2 days after administration, and 1: about 3.5 to about 4.2, 1: about 3.7 to 3.9, or 1: about 3.8 to about 3.9 at about 7 days after administration, but is not limited thereto.

The distribution ratio in the liver relative to the heart after administration may be one of the followings:
(a) a distribution ratio in the liver relative to the heart of 1: 1.5 to 3.0 at 2 hours to 5 hours after administration;
(b) a distribution ratio in the liver relative to the heart of 1: 2.0 to 3.0 at 40 hours to 50 hours after administration; and
(c) a distribution ratio in the liver relative to the heart of 1: 5.5 to 7.0 at 160 hours to 180 hours after administration, but is not limited thereto.

The distribution ratio in the liver relative to the heart after administration may be one of the followings:
(a) a distribution ratio in the liver relative to the heart of 1: about 1.5 to about 3.0, 1: about 1.8 to about 3.0, 1: about 2.0 to about 2.8, 1: about 2.1 to about 2.5, or 1: about 2.2 to about 2.8 at 2 hours to 5 hours after administration;
(b) a distribution ratio in the liver relative to the heart of 1: about 1.5 to about 3.0, 1: about 1.8 to about 3.0, 1: about 2.0 to about 2.8, 1: about 2.1 to about 2.5, 1: about 2.2 to about 2.8 at 2 hours to 5 hours after administration, and 1: about 2.0 to 3.0, 1: about 2.4 to 3.0, 1: about 2.5 to 3.0, 1: about 2.6 to 3.0, 1: about 2.7 to 3.0, 1: about 2.8 to about 3.0, or 1: about 2.8 to 2.9 at about 40 hours to about 50 hours, about 45 hours to about 50 hours, or about 2 days after administration; and
(c) a distribution ratio in the liver relative to the heart of 1: about 5.5 to about 7.0, 1: about 5.8 to about 7.0, 1: about 5.9 to about 7.0, 1: about 6.0 to about 7.0, 1: about 6.1 to about 7.0, 1: about 6.2 to about 7.0, 1: about 6.3 to about 7.0, 1: about 6.4 to about 7.0, 1: about 6.5 to about 7.0, 1: about 6.5 to about 6.9, 1: about 6.5 to about 6.8, 1: about 6.5 to about 6.7, 1: about 6.5 to about 6.6, or 1: about 6.55 to about 6.6 at about 160 hours to about 180 hours, about 160 hours to about 180 hours, or about 7 days after administration, but is not limited thereto.

The distribution ratio may be measured based on T/S (%), and when the T/S (%) of the heart is set as 1, the multiple of T/S (%) in the liver may be confirmed.

As used herein, the term "about" refers to a range which includes all of ±0.5, ±0.4, ±0.3, ±0.2, ±0.1, *etc.* and includes all of the values that are equivalent or similar to those following the values, but the range is not limited thereto.

The liver-targeted drug may include a substance that can be targeted to the liver tissue. Specifically, the liver-targeted drug may bind to a glucagon receptor capable of inducing targeting of the drug to the liver and may include a peptide sequence having activity on a glucagon receptor, a GLP-1 receptor, and a GIP receptor. For example, it may be a peptide including the amino acid sequence of Formula 1 or a long-acting conjugate containing the same, but is not limited thereto. The peptide may be a peptide having activity on a glucagon receptor, a GLP-1 receptor, and a GIP receptor, but is not limited thereto.

Meanwhile, as used herein, the phrase "having a binding affinity to a glucagon receptor" means that a liver-targeted drug has a binding affinity to a glucagon receptor to such an extent that it can be targeted to the liver from the blood when the drug is delivered into the blood of an animal. More specifically, it means that the distribution ratio of the drug in the liver is higher by about 5% or more, about 10% or more, about 15% or more, about 20% or more, about 30 % or more, or about 40% or more relative to at least one other organ such as the heart, lung, large intestine, spleen, pancreas, adipose tissue, small intestine, stomach, muscle, kidney, or brain, when the distribution ratio of the drug in the organs was confirmed at least 1 hour, at least 4 hours, at least 48 hours, or at least 168 hours after the drug was delivered into the animal's blood, but is not particularly limited thereto.

The "peptide having activity on a glucagon receptor, a GLP-1 receptor, and a GIP receptor" may be used interchangeably with a triple agonist or a triple agonist peptide in the present invention.

Such a peptide may include various substances, *e.g.,* various peptides, which have a significant level of activity on glucagon, GLP-1, and GIP receptors.

Although not particularly limited, the triple agonist having a significant level of activity on glucagon, GLP-1, and GIP receptors may exhibit *in vitro* activity of about 0.01% or higher, about 0.1% or higher, about 1% or higher, about 2% or higher, about 3 % or higher, about 4% or higher, about 5% or higher, about 6% or higher, about 7% or higher, about 8% or higher, about 9% or higher, about 10 % or higher, about 20% or higher, about 30% or higher, about 40% or higher, about 50 % or higher, about 60% or higher, about 70% or higher, about 80% or higher, about 90% or higher, or about 100% or higher on one or more receptors, specifically two or more receptors, and more specifically all three of the receptors among the glucagon, GLP-1, GIP receptors, relative to that of native ligands (native glucagon, native GLP-1, and native GIP) of the corresponding receptors.

More specifically, the triple agonist may exhibit *in vitro* activity on a glucagon receptor of about 0.01% or higher, about 0.1% or higher, about 1% or higher, about 2% or higher, about 3 % or higher, about 4% or higher, about 5% or higher, about 6% or higher, about 7% or higher, about 8% or higher, about 9% or higher, about 10 % or higher, about 20% or higher, about 30% or higher, about 40% or higher, about 50 % or higher, about 60% or higher, about 70% or higher, about 80% or higher, about 90% or higher, or about 100% or higher, relative to that of a native ligand (native glucagon), but is not limited thereto.

The *in vitro* activity of the triple agonist may be measured with reference to Experimental Example 1, but the method is not particularly limited thereto.

Meanwhile, the peptide is characterized in that it exhibits one or more, two or more, specifically all three activities from i) to iii) below, in particular a significant activity:
i) activation of glucagon receptor; ii) activation of GLP-1 receptor; and iii) activation of GIP receptor.

More specifically, the peptide may exhibit activity on a GLP-1 receptor and/or activity on a GIP receptor, while exhibiting activity on a glucagon receptor, but is not particularly limited thereto.

In particular, the activation of the receptors may include, for example, those cases where the *in vitro* activity is about 0.1% higher, about 1% or higher, about 2% or higher, about 3 % or higher, about 4% or higher, about 5% or higher, about 6% or higher, about 7% or higher, about 8% or higher, about 9% or higher, about 10 % or higher, about 20% or higher, about 30% or higher, about 40% or higher, about 50 % or higher, about 60% or higher, about 70% or higher, about 80% or higher, about 90 % or higher, and about 100% or higher, relative to native receptors, but is not limited thereto.

Additionally, the peptide may be one which has an increased *in vivo* half-life compared to any one of native glucagon, native GLP-1, and native GIP, but is not particularly limited thereto.

Although not particularly limited, the peptide may be one which is non-naturally occurring.

Meanwhile, although not particularly limited, the native glucagon may have the following amino acid sequence:

His-Ser-Gln-Gly-Thr-Phe-Thr-Ser-Asp-Tyr-Ser-Lys-Tyr-Leu-Asp-Ser-Arg-Arg-Ala
-Gln-Asp-Phe-Val-Gln-Trp-Leu-Met-Asn-Thr (SEQ ID NO: 118)

Specifically, the peptide may be an analog of native glucagon in which at least one amino acid in the native glucagon sequence is modified through substitution, addition, deletion, modification, and a combination thereof, but if the peptide has a high distribution in the liver of the body organs of an administered subject, it may fall within the scope of the present invention.

In addition, the substitution of the amino acids may include both substitution of amino acids or substitution with non-native compounds.

Examples of the glucagon analog prepared by a combination of these methods may include peptides, whose amino acid sequences differ from that of native glucagon in at least one amino acid, and in which the α-carbon of the amino acid residue in the N-terminus is removed, while having activities on a glucagon receptor, a GLP-1 receptor, and a GIP receptor, *etc.,* but are not limited thereto, and analogs of native glucagon applicable to the present invention may be prepared by a combination of various methods for the preparation of analogs.

Additionally, although not particularly limited, a part of the amino acids of the peptide may be substituted with other amino acids or non-native compounds to avoid recognition by a degradation enzyme of the triple agonist for increasing the *in vivo* half-life.

Specifically, the triple agonist may be a peptide in which the *in vivo* half-life is increased by avoiding recognition by the degradation enzyme through a substitution of the 2^{nd} amino acid sequence among the amino acid sequences of the triple agonist, but any substitution or modification of amino acids to avoid recognition by an *in vivo* degradation enzyme is included without limitation.

Additionally, such modification for preparing analogs of native glucagon may include all of the modifications using L-type or D-type amino acids and/or non-natural amino acids; and/or a modification of the native sequence, for example, a modification of a side chain functional group, an intramolecular covalent bonding (*e.g.,* a ring formation between side chains), methylation, acylation, ubiquitination, phosphorylation, aminohexanation, biotinylation, *etc.*

The modification also includes all of the additions of one or more amino acids to the amino and/or carboxy terminus of native glucagon.

As the amino acids for the substitution or addition, not only the 20 amino acids commonly found in human proteins, but also atypical or non-naturally occurring amino acids may be used. Commercial sources of atypical amino acids may include Sigma-Aldrich, ChemPep Inc., and Genzyme Pharmaceuticals. The peptides including these amino acids and typical peptide sequences may be synthesized and purchased from commercial peptide synthesis companies, *e.g.,* American Peptide Company, Bachem (USA), or Anygen (Korea).

Amino acid derivatives may be obtained in the same manner, for example, 4-imidazoacetic acid, *etc.* may be used.

In one specific embodiment, the peptide having activity on a glucagon receptor, GLP-1 receptor, and a GIP receptor, the liver-targeted drug, may include the amino acid sequence represented by General Formula 1 below: 
Xaa1-Xaa2-Xaa3-Gly-Thr-Phe-Xaa7-Ser-Asp-Xaa10-Ser-Xaa12-Xaa13-Xaa14-
Xaa15-Xaa16-Xaa17-Xaa18-Xaa19-Xaa20-Xaa21-Phe-Xaa23-Xaa24-Trp-Leu-Xaa27-X
aa28-Xaa29-Xaa30-R1 (General Formula 1, SEQ ID NO: 103)
 wherein in the General Formula 1 above,
Xaa1 is histidine, 4-imidazoacetyl, or tyrosine;
Xaa2 is glycine, α-methyl-glutamic acid, or Aib;
Xaa3 is glutamic acid or glutamine;
Xaa7 is threonine or isoleucine;
Xaa10 is leucine, tyrosine, lysine, cysteine, or valine;
Xaa12 is lysine, serine, or isoleucine;
Xaa13 is glutamine, tyrosine, alanine, or cysteine;
Xaa14 is leucine, methionine, or tyrosine;
Xaa15 is cysteine, aspartic acid, glutamic acid, or leucine;
Xaa16 is glycine, glutamic acid, or serine;
Xaa17 is glutamine, arginine, isoleucine, glutamic acid, cysteine, or lysine;
Xaa18 is alanine, glutamine, arginine, or histidine;
Xaa19 is alanine, glutamine, cysteine, or valine;
Xaa20 is lysine, glutamine, or arginine;
Xaa21 is glutamic acid, glutamine, leucine, cysteine, or aspartic acid;
Xaa23 is isoleucine or valine;
Xaa24 is alanine, glutamine, cysteine, asparagine, aspartic acid, or glutamic acid;
Xaa27 is valine, leucine, lysine, or methionine;
Xaa28 is cysteine, lysine, alanine, asparagine, or aspartic acid;
Xaa29 is cysteine, glycine, glutamine, threonine, glutamic acid, or histidine;
Xaa30 is cysteine, glycine, lysine, or histidine, or is absent; and
R1 is cysteine (Cys, C), GKKNDWKHNIT (SEQ ID NO: 106), m-SSGAPPPS-n (SEQ ID NO: 107), or m-SSGQPPPS-n (SEQ ID NO: 108), or is absent;
   wherein,
   m is Cys, Pro, or Gly-Pro;
   n is Cys, Gly, Ser, or His-Gly, or is absent.

For example, the triple agonist may be one which includes an amino acid sequence selected from the group consisting of SEQ ID NOS: 1 to 102; or one which consists (essentially) of an amino acid sequence selected from the group consisting of SEQ ID NOS: 1 to 102, but is not limited thereto.

Additionally, although described as 'a peptide consisting of a particular SEQ ID NO' in the present invention, it does not exclude a mutation that may occur by the addition of a meaningless sequence upstream or downstream of the amino acid sequence of the corresponding SEQ ID NO, or a mutation that may occur naturally, or a silent mutation thereof, as long as the peptide has an activity the same as or corresponding to that of the peptide which consists of an amino acid sequence of the corresponding SEQ ID NO, and it obviously belongs to the scope of the present invention even when the peptide has such a sequence addition or mutation therein. That is, the peptide may belong to the scope of the present invention as long as it exhibits a certain level of homology or identity even when a difference in sequences is present, and has a high distribution in the liver among the body organs of an administered subject.

For example, a peptide consisting of a particular SEQ ID NO, or a peptide having at least 60% or higher, 65% or higher, 70% or higher, 75% or higher, 80% or higher, 85% or higher, 90% or higher, 91% or higher, 92% or higher, 93% or higher, 94% or higher, or 95% or higher sequence homology to the peptide including a particular SEQ ID NO may also be included, and when the peptide has a high distribution in the liver of the peptide among the body organs of an administered subject, it is not limited to a specific sequence.

As used herein, the term 'homology' or 'identity' refers to the degree of relatedness between two given amino acid sequences or nucleotide sequences, and may be expressed as a percentage. The terms homology and identity may be often used interchangeably with each other.

Whether any two peptide sequences have homology, similarity, or identity may be determined by a known computer algorithm such as the "FASTA" program using default parameters as in Pearson et al. (1988) Proc. Natl. Acad. Sci. USA 85:2444. Alternatively, it may be determined by the Needleman-Wunsch algorithm (Needleman and Wunsch, 1970, J. Mol. Biol. 48:443-453), which is performed using the Needleman program of the EMBOSS package (EMBOSS: The European Molecular Biology Open Software Suite, Rice et al., 2000, Trends Genet. 16:276-277) (version 5.0.0 or later) (GCG program package (Devereux, J. et at, Nucleic Acids Research 12:387 (1984)), BLASTP, BLASTN, FASTA (Atschul, S. F. et al., J MOLEC BIOL 215:403 (1990); Guide to Huge Computers, Martin J. Bishop, ed., Academic Press, San Diego, 1994, and CARILLO et al. (1988) SIAM J Applied Math 48:1073). For example, homology, similarity, or identity may be determined using BLAST or ClustalW of the National Center for Biotechnology Information.

The homology, similarity, or identity of peptides may be determined by comparing sequence information using, for example, the GAP computer program, such as Needleman et al. (1970), J Mol Biol. 48:443, as disclosed in Smith and Waterman, Adv. Appl. Math (1981) 2:482. In summary, the GAP program defines the homology, similarity or identity as the value obtained by dividing the number of similarly aligned symbols (*i.e.*, nucleotides or amino acids) by the total number of the symbols in the shorter of the two sequences. Default parameters for the GAP program may include (1) a binary comparison matrix (containing a value of 1 for identities and 0 for non-identities) and the weighted comparison matrix (or EDNAFULL (EMBOSS version of NCBI NUC4.4) substitution matrix) of Gribskov et al. (1986) Nucl Acids Res. 14:6745, as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or a gap opening penalty of 10 and a gap extension penalty of 0.5); and (3) no penalty for end gaps. Therefore, as used herein, the term "homology" or "identity" refers to the relevance between sequences.

The above disclosure may be applied to other embodiments or other aspects of the present invention, but is not limited thereto.

In one embodiment, in the General Formula 1 above, Xaa14 may be leucine or methionine; and Xaa15 may be cysteine, aspartic acid, or leucine.

Examples of the peptide may include a peptide including an amino acid sequence selected from the group consisting of SEQ ID NO: 1 to 12, 14 to 17, and 21 to 102, or a peptide consisting (essentially) of the same, but are not particularly limited thereto.

The peptide may significantly activate one or more of the glucagon receptor, GLP-1 receptor, and GIP receptor, but is not particularly limited thereto. Specifically, the peptide may be one which significantly activates the GLP-1 receptor, or additionally the glucagon receptor and/or GIP receptor, but is not particularly limited thereto.

In another example,
in the General Formula 1 above,
Xaa2 may be glycine, α-methyl-glutamic acid, or Aib;
Xaa7 may be threonine;
Xaa10 may be tyrosine, cysteine, or valine;
Xaa12 may be lysine or isoleucine;
Xaa13 may be tyrosine, alanine, glutamine, or cysteine;
Xaa14 may be leucine, tyrosine, or methionine;
Xaa15 may be cysteine, leucine, glutamic acid, or aspartic acid;
Xaa17 may be glutamine, arginine, isoleucine, cysteine, glutamic acid, or lysine;
Xaa18 may be alanine, glutamine, arginine, or histidine;
Xaa19 may be alanine, glutamine, valine, or cysteine;
Xaa20 may be lysine, arginine, or glutamine;
Xaa21 may be glutamic acid, glutamine, leucine, cysteine, or aspartic acid;
Xaa23 may be isoleucine or valine;
Xaa24 may be cysteine, alanine, glutamine, asparagine, glutamic acid, or aspartic acid; and
Xaa27 may be leucine or lysine, but is not particularly limited thereto.

In still another example,
in the General Formula 1 above,
Xaa2 may be glycine, α-methyl-glutamic acid, or Aib;
Xaa7 may be threonine;
Xaa10 may be tyrosine, cysteine, or valine;
Xaa12 may be lysine or isoleucine;
Xaa13 may be tyrosine, alanine, or cysteine;
Xaa14 may be leucine or methionine;
Xaa15 may be cysteine or aspartic acid;
Xaa17 may be glutamine, arginine, isoleucine, cysteine, or lysine;
Xaa18 may be alanine, arginine, or histidine;
Xaa19 may be alanine, glutamine, or cysteine;
Xaa20 may be lysine or glutamine;
Xaa21 may be glutamic acid, cysteine, or aspartic acid;
Xaa23 may be valine;
Xaa24 may be alanine, glutamine, cysteine, asparagine, or aspartic acid; and
Xaa27 may be leucine or lysine, but is not particularly limited thereto.

In yet another example,
in the General Formula 1 above,
Xaa2 may be α-methyl-glutamic acid or Aib;
Xaa7 may be threonine;
Xaa10 may be tyrosine or cysteine;
Xaa12 may be lysine or isoleucine;
Xaa13 may be tyrosine, alanine, or cysteine;
Xaa14 may be leucine or methionine;
Xaa15 may be cysteine or aspartic acid;
Xaa16 may be glutamic acid;
Xaa17 may be arginine, isoleucine, cysteine, or lysine;
Xaa18 may be alanine, arginine, or histidine;
Xaa19 may be alanine, glutamine, or cysteine;
Xaa20 may be lysine or glutamine;
Xaa21 may be glutamic acid or aspartic acid;
Xaa23 may be valine;
Xaa24 may be glutamine, asparagine, or aspartic acid;
Xaa27 may be leucine; and
Xaa28 may be cysteine, alanine, asparagine, or aspartic acid.

In even another example,
in the General Formula 1 above,
Xaa1 may be histidine or 4-imidazoacetyl;
Xaa2 may be α-methyl-glutamic acid or Aib;
Xaa3 may be glutamine;
Xaa7 may be threonine;
Xaa10 may be tyrosine;
Xaa12 may be isoleucine,
Xaa13 may be alanine or cysteine;
Xaa14 may be methionine,
Xaa15 may be aspartic acid;
Xaa16 may be glutamic acid;
Xaa17 may be isoleucine or lysine;
Xaa18 may be alanine or histidine;
Xaa19 may be glutamine or cysteine;
Xaa20 may be lysine;
Xaa21 may be aspartic acid;
Xaa23 may be valine,
Xaa24 may be asparagine;
Xaa27 may be leucine;
Xaa28 may be alanine or asparagine;
Xaa29 may be glutamine or threonine; and
Xaa30 may be cysteine or lysine, or is absent.

In further another example,
in the General Formula 1 above,
Xaa2 may be glycine, α-methyl-glutamic acid, or Aib;
Xaa3 may be glutamine;
Xaa7 may be threonine;
Xaa10 may be tyrosine, cysteine, or valine;
Xaa12 may be lysine;
Xaa13 may be tyrosine;
Xaa14 may be leucine;
Xaa15 may be aspartic acid;
Xaa16 may be glycine, glutamic acid, or serine;
Xaa17 may be glutamine, arginine, cysteine, or lysine;
Xaa18 may be alanine, arginine, or histidine;
Xaa19 may be alanine or glutamine;
Xaa20 may be lysine or glutamine;
Xaa21 may be glutamic acid, cysteine, or aspartic acid;
Xaa23 may be valine;
Xaa24 may be alanine, glutamine, or cysteine;
Xaa27 may be leucine or lysine;
Xaa29 may be glycine, glutamine, threonine, or histidine, but is not particularly limited thereto.

These peptides may correspond to a case where the peptide has significant activation levels on the GLP-1 receptor and glucagon receptor, and higher activation levels compared to that on the GIP receptor; a case where the peptide has significant activation levels on all of the GLP-1 receptor, glucagon receptor, and GIP receptor; or a case where the peptide has significant activation levels on the GLP-1 receptor and GIP receptor and higher activation levels compared to that on the glucagon receptor; but are not particularly limited thereto.

When the peptide has significant activation levels on the GLP-1 receptor and GIP receptor, and higher activation levels compared to that on the glucagon receptor, a peptide with more improved capability of controlling blood glucose levels along with the effect of reducing body weight may be provided. In contrast, when the peptide has significant activation levels on all of the GLP-1 receptor, glucagon receptor, and GIP receptor, there is an advantage in that the effect of reducing body weight can be maximized, but is not particularly limited thereto, and any peptide may fall within the scope of the present invention as long as it can be targeted to the liver, and optionally exhibits therapeutic activity on the liver.

Examples of the peptide may include a peptide including an amino acid sequence selected from the group consisting of SEQ ID NOS: 8, 9, 21 to 37, 39, 42, 43, 49 to 61, 64 to 83, 85, 86, 88, 89, 91 to 93, and 95 to 102; or a peptide consisting (essentially) of the same, but are not particularly limited thereto.

In one embodiment, the peptide, which is the liver-targeted drug, may include the amino acid sequence represented by General Formula 2 below: 
Xaa1-Xaa2-Gln-Gly-Thr-Phe-Thr-Ser-Asp-Xaa10-Ser-Lys-Xaa13-Xaa14-Xaa15-
Xaa16-Xaa17-Xaa18-Xaa19-Xaa20-Xaa21-Phe-Xaa23-Xaa24-Trp-Leu-Leu-Xaa28-Xaa
29-Xaa30-Xaa31- Ser-Ser-Gly-Gln-Pro-Pro-Pro-Ser-Xaa40 (General Formula 2, SEQ ID
NO: 104)
 in the General Formula 2 above,
Xaa1 is 4-imidazoacetyl, histidine, or tyrosine;
Xaa2 is glycine, α-methyl-glutamic acid, or Aib;
Xaa10 is tyrosine or cysteine;
Xaa13 is alanine, glutamine, tyrosine, or cysteine;
Xaa14 is leucine, methionine, or tyrosine;
Xaa15 is aspartic acid, glutamic acid, or leucine;
Xaa16 is glycine, glutamic acid, or serine;
Xaa17 is glutamine, arginine, isoleucine, glutamic acid, cysteine, or lysine;
Xaa18 is alanine, glutamine, arginine, or histidine;
Xaa19 is alanine, glutamine, cysteine, or valine;
Xaa20 is lysine, glutamine, or arginine;
Xaa21 is cysteine, glutamic acid, glutamine, leucine, or aspartic acid;
Xaa23 is isoleucine or valine;
Xaa24 is cysteine, alanine, glutamine, asparagine, or glutamic acid;
Xaa28 is lysine, cysteine, asparagine, or aspartic acid;
Xaa29 is glycine, glutamine, cysteine, or histidine;
Xaa30 is cysteine, glycine, lysine, or histidine;
Xaa31 is proline or cysteine; and
Xaa40 is cysteine, or is absent.

More specifically, in the General Formula 2 above,
Xaa13 may be alanine, tyrosine, or cysteine;
Xaa15 may be aspartic acid or glutamic acid;
Xaa17 may be glutamine, arginine, cysteine, or lysine;
Xaa18 may be alanine, arginine, or histidine;
Xaa21 may be cysteine, glutamic acid, glutamine, or aspartic acid;
Xaa23 may be isoleucine or valine;
Xaa24 may be cysteine, glutamine, or asparagine;
Xaa28 may be cysteine, asparagine, or aspartic acid;
Xaa29 may be glutamine, cysteine, or histidine; and
Xaa30 may be cysteine, lysine, or histidine.

Examples of the peptide may include a peptide including an amino acid sequence selected from the group consisting of SEQ ID NOS: 21, 22, 42, 43, 50, 64 to 77, and 95 to 102, more specifically, a peptide including an amino acid sequence selected from the group consisting of SEQ ID NOS: 21, 22, 42, 43, 50, 64 to 77, and 96 to 102; or a peptide consisting (essentially) of the same, but are not particularly limited thereto.

In one embodiment, the peptide, which is the liver-targeted drug, may include the amino acid sequence represented by General Formula 3 below: 
Xaa1-Xaa2-Gln-Gly-Thr-Phe-Thr-Ser-Asp-Tyr-Ser-Lys-Xaa13-Leu-Asp-Glu-Xaa
17-Xaa18-Xaa19-Lys-Xaa21-Phe-Val-Xaa24-Trp-Leu-Leu-Xaa28-Xaa29-Xaa30-Xaa31-
Ser-Ser-Gly-Gln-Pro-Pro-Pro-Ser-Xaa40 (General Formula 3, SEQ ID NO: 105),
 wherein in the General Formula 3 above,
Xaa1 is histidine or tyrosine;
Xaa2 is α-methyl-glutamic acid or Aib;
Xaa13 is alanine, tyrosine or cysteine;
Xaa17 is arginine, cysteine, or lysine;
Xaa18 is alanine or arginine;
Xaa19 is alanine or cysteine;
Xaa21 is glutamic acid or aspartic acid;
Xaa24 is glutamine or asparagine;
Xaa28 is cysteine or aspartic acid;
Xaa29 is cysteine, histidine, or glutamine;
Xaa30 is cysteine or histidine;
Xaa31 is proline or cysteine; and
Xaa40 is cysteine, or is absent.

Examples of the peptide may include a peptide including an amino acid sequence selected from the group consisting of SEQ ID NOS: 21, 22, 42, 43, 50, 64 to 71, 75 to 77, and 96 to 102; or a peptide consisting (essentially) of the same, but are not particularly limited thereto.

Additionally, in the General Formula 1 above, R1 may be cysteine, GKKNDWKHNIT (SEQ ID NO: 106), CSSGQPPPS (SEQ ID NO: 109), GPSSGAPPPS (SEQ ID NO: 110), GPSSGAPPPSC (SEQ ID NO: 111), PSSGAPPPS (SEQ ID NO: 112), PSSGAPPPSG (SEQ ID NO: 113), PSSGAPPPSHG (SEQ ID NO: 114), PSSGAPPPSS (SEQ ID NO: 115), PSSGQPPPS (SEQ ID NO: 116), or PSSGQPPPSC (SEQ ID NO: 117), or may be absent, but is not particularly limited thereto.

The triple agonist peptide described above may include an intramolecular bridge (*e.g.,* a covalent bridge or a non-covalent bridge), and specifically, may be in a form including a ring. For example, the triple agonist peptide may be in a form where a ring is formed between the amino acids at positions 16 and 20 of the triple agonist peptide, but is not particularly limited thereto.

Non-limiting examples of the ring may include a lactam bridge (or lactam ring).

Additionally, the triple agonist peptide includes all of those which are modified to include an amino acid capable of forming a ring at the desired site so as to include a ring.

Such a ring may be formed between side chains of amino acids within the triple agonist peptide, *e.g.,* the ring may be in a form where a lactam ring is formed between a side chain of lysine and a side chain of a glutamic acid), but is not particularly limited thereto.

For example, the peptide including the amino acid sequence of General Formulae 1 to 3 above may be one in which each amino acid in each acid pair among the amino acid pairs of Xaa10 and Xaa14, Xaa12 and Xaa16, Xaa16 and Xaa20, Xaa17 and Xaa21, Xaa20 and Xaa24, and Xaa24 and Xaa28 of General Formulae 1 to 3 may be substituted with glutamic acid or lysine, but is not limited thereto. In the Xaan (n is a natural number), n refers to the position of the amino acid from the N-terminus of an amino acid sequence provided.

Additionally, the peptide including the amino acid sequence of General Formulae 1 to 3 above may be one in which each amino acid in each amino acid pair of Xaa12 and Xaa16, the amino acid pair of Xaa16 and Xaa20, or the amino acid pair of Xaa17 and Xaa21 may be substituted with glutamic acid or lysine, which is capable of forming a ring, but is not limited thereto.

Additionally, in the General Formulae 1 to 3 above, the peptide may be one in which a ring (*e.g.,* a lactam ring) is formed between each amino acid in each amino acid pair in at least one amino acid pair among the amino acid pairs of Xaa10 and Xaa14, Xaa12 and Xaa16, Xaa16 and Xaa20, Xaa17 and Xaa21, Xaa20 and Xaa24, and Xaa24 and Xaa28, but is not limited thereto.

Additionally, in the General Formulae 1 to 3 above, Xaa16 may be glutamic acid, Xaa20 may be lysine, and the side chains of X16 and X20 may form a lactam ring, but is not limited thereto.

Additionally, the peptide according to the present invention may be in a form where the N-terminus and/or C-terminus is not modified, however, those variants where the N-terminus and/or C-terminus, *etc.,* of the peptide are chemically modified or protected by organic groups, or where amino acids are added to the end of the peptide for its protection from proteases *in vivo* while increasing its stability, may also be included in the scope of the peptides of the present invention. In a case where the C-terminus is not modified, the end of the peptide according to the present invention may have a carboxyl group, but is not particularly limited thereto.

In particular, in the case of a chemically-synthesized peptide, its N- and C-termini are electrically charged, and thus the N-terminus may be acetylated and/or the C-terminus may be amidated in order to remove the charge, but the peptide is not particularly limited thereto.

Unless specified otherwise in the present specification, the description in the detailed description or claims with respect to "peptide" according to the present invention or a "conjugate", in which such a peptide is covalently linked to an immunoglobulin Fc fragment, may be applied to the forms, which include not only the corresponding peptide or conjugate, but also salts of the corresponding peptide or conjugate (*e.g.,* pharmaceutically acceptable salts thereof), or solvates thereof. Accordingly, the description of the "peptide" or "conjugate" throughout the specification may also be equally applied to a particular salt thereof, a particular solvate thereof, and a particular solvate of the particular salt thereof. These salts may be, for example, in a form where any pharmaceutically acceptable salts are used.

The kind of the salt is not particularly limited. However, the salt is preferably one that is safe and effective to a subject, *e.g.,* a mammal, but is not particularly limited thereto.

The term "pharmaceutically acceptable" refers to a material which can be effectively used for the intended use within the scope of pharmaco-medical decision without inducing excessive toxicity, irritation, allergic responses, *etc.*

As used herein, the term "pharmaceutically acceptable salt" refers to a salt derived from pharmaceutically acceptable inorganic salts, organic salts, or bases. Examples of the suitable salts may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, benzenesulfonic acid, *etc.* Examples of the salts derived from suitable bases may include alkali metals such as sodium, potassium, *etc*.; alkali earth metals such as magnesium; and ammonium, *etc.*

Additionally, as used herein, the term "solvate" refers to a complex formed between the peptide, conjugate according to the present invention or a salt thereof, and a solvent molecule.

The C-terminus of the peptide according to the present invention may be an amidated peptide or a peptide having a free carboxyl group (-COOH), or may include a peptide having an unmodified C-terminus, but is not limited thereto.

In one embodiment, the peptide may be amidated at the C-terminus, but is not limited thereto.

In one embodiment, the peptide may be non-glycosylated, but is not limited thereto.

The peptide of the present invention may be synthesized through a solid phase synthesis method, may be produced by a recombinant method, and may be produced commercially, but is not limited thereto.

Additionally, the peptide of the present invention may be synthesized according to its length by a method well known in the art (*e.g.,* by an automatic peptide synthesizer), and may also be produced by genetic engineering technology.

Specifically, the peptide of the present invention may be prepared by a standard synthesis method, a recombinant expression system, or any other method known in the art. Accordingly, the peptide of the present invention may be synthesized by various methods including, for example, the methods described below:
(a) a method of synthesizing a peptide by a solid-phase or liquid-phase method stepwise or by fragment assembly, followed by isolation and purification of the final peptide product; or
(b) a method of expressing a nucleic acid construct encoding a peptide in a host cell and recovering the expression product from the host cell culture;
(c) a method of performing an *in vitro* cell-free expression of a nucleic acid construct encoding a peptide and recovering the expression product therefrom; or
a method of obtaining peptide fragments by any combination of the methods (a), (b), and (c), obtaining the peptide by linking the peptide fragments, and then recovering the peptide.

Additionally, the peptide having activity on the glucagon receptor, GLP-1 receptor, and GIP receptor may be in the form of a long-acting conjugate in which a biocompatible material moiety capable of increasing the *in vivo* half-life thereof is linked to the peptide having activity on the glucagon receptor, GLP-1 receptor, and GIP receptor, but is not limited thereto. The biocompatible material moiety may be interchangeably used with a carrier in the present specification.

The peptide (in the form of a conjugate) including the amino acid sequences of General Formulae 1 to 3 in the form of the long-acting conjugate, or the peptide including the amino acid sequences of General Formula 1 to 3 itself, which is the liver targeted drug, is an active ingredient of a pharmaceutical composition which has a high distribution of the drug in the liver among the body organs of an administered subject.

As used herein, the term "long-acting conjugate", which is in the form where a biocompatible material moiety or carrier is linked to a physiologically active material (*e.g.,* a triple agonist peptide), may specifically include a peptide moiety and a biocompatible material moiety which is covalently linked to the peptide moiety, wherein the peptide moiety may be represented by the amino acid sequences of General Formulae 1 to 3 above, or may be a sequence identical to any one sequence selected from SEQ ID NOS: 1 to 102, or a sequence including the same. In the long-acting conjugate, the biocompatible material moiety or carrier may be one that is covalently linked to the physiologically active material, but is not particularly limited thereto. The C-terminus of the peptide of the amino acid sequences of General Formulae 1 to 3 above, the sequence identical to any one sequence selected from SEQ ID NOS: 1 to 102, or the sequence including the same may be an amidated peptide or a peptide having a free carboxyl group (-COOH), or may include a peptide having an unmodified C-terminus, but is not limited thereto.

In the present invention, the conjugate of the peptide may exhibit an increased efficacy of duration and/or an increased serum half-life, compared to that of a peptide to which a carrier is not linked, and as used herein, such a conjugate is referred to as a "long-acting conjugate".

Meanwhile, the conjugate may be non-naturally occurring.

In a specific embodiment of the present invention, the long-acting conjugate may refer to a form in which an immunoglobulin Fc region and the triple agonist peptide are linked to each other. Specifically, the conjugate may be one in which an immunoglobulin Fc region is covalently linked to the triple agonist peptide through a linker, but is not particularly limited thereto.

In one embodiment, the immunoglobulin Fc region and X may not be glycosylated, but is not limited thereto.

In one embodiment of the present invention, the long-acting conjugate may be a conjugate represented by Formula 1 below:

[Formula 1] X-L-F

wherein,
X is the peptide above;
L is a linker containing an ethylene glycol repeating unit;
F is an immunoglobulin Fc region; and
- represents a covalent bond between X and L, and between L and F.

The X of the long-acting conjugate of Formula 1 above may be the triple agonist peptide described above, but is not limited thereto.

In one embodiment, the X of the long-acting conjugate of Formula 1 above may be a peptide including the amino acid sequence of General Formula 1 above.

In one embodiment, the X of the long-acting conjugate of Formula 1 above may be a peptide including the amino acid sequence of General Formula 2 or 3 above.

Specifically, the X may be a peptide including an amino acid sequence of any one SEQ ID NO selected from the group consisting of SEQ ID NOS: 1 to 102, but is not limited thereto.

In the conjugate, F is X, the liver-targeted drug, specifically a drug including a substance having a binding affinity to a glucagon receptor, for example, a peptide having activity on a glucagon receptor, a GLP-1 receptor, and a GIP receptor, and corresponds to one constitution of the moiety constituting the conjugate of the present invention. The liver-targeted drug is as described above.

The F and X may be linked to each other by a covalent chemical bond or a non-covalent chemical bond, and the F and X may be linked to each other through L by a covalent chemical bond, a non-covalent chemical bond, or a combination thereof.

As used herein, the term "long-acting conjugate of Formula 1" which is in the form where the triple agonist peptide and an immunoglobulin Fc region are linked to through a linker, may exhibit an increased efficacy of duration compared to a triple agonist peptide to which an immunoglobulin Fc region is not linked.

In the long-acting conjugate, F is a substance capable of increasing the half-life of X, i.e., the triple agonist peptide, and corresponds to one constitution of the moiety constituting the conjugate of the present invention.

In the long-acting conjugate of Formula 1, the linkage between X, the peptide, which is the triple agonist, and the immunoglobulin Fc region may be achieved by a physical or chemical bond, or a non-covalent or covalent bond, and specifically a covalent bond, but is not limited thereto.

Additionally, the method of linking X, the triple agonist peptide of the long-acting conjugate of Formula 1, and the immunoglobulin Fc region is not particularly limited, but the triple agonist peptide and the immunoglobulin Fc region may be linked to each other through a linker.

In Formula 1 above, X and F may be linked to each other through L by a covalent bond.

More specifically, X and L, and L and F may be linked to each other by a covalent bond, and in particular, the conjugate may be a conjugate in which X, L, and F are each linked by a covalent bond in the order of Formula 1.

Additionally, the X may be linked to F through a linker (L).

Meanwhile, the L may be a non-peptidyl linker, *e.g.,* a linker containing an ethylene glycol repeating unit.

In the present invention, the "non-peptidyl linker" includes a biocompatible polymer in which two or more repeating units are linked. The repeating units are linked to each other through any covalent bond which is not a peptide bond. The non-peptidyl linker may be one constitution constituting the moiety of the conjugate of the present invention, and corresponds to L in Formula 1 above. As the non-peptidyl linker that can be used herein, any polymer which has a resistance to proteases *in vivo* can be used without limitation. In the present invention, the non-peptidyl linker may be used interchangeably with a non-peptidyl polymer.

Although not particularly limited, the non-peptide linker may be a linker containing an ethylene glycol repeating unit (*e.g.,* polyethylene glycol), and additionally, derivatives thereof which are already known in the art and derivatives that can be easily prepared at the technological level of those skilled in the art are included in the scope of the present invention.

The repeating unit of the non-peptide linker may be an ethylene glycol repeating unit, and specifically, the non-peptide linker may be one which includes a functional group used for the preparation of a conjugate at an end before being formed into a conjugate, while including an ethylene glycol repeating unit. The long-acting conjugate according to the present invention may be in the form in which X and F are linked through the functional group, but is not limited thereto. In the present invention, the non-peptide linker may include two, or three or more functional groups, and each functional group may be the same as or different from each other, but the non-peptide linker is not limited thereto.

Specifically, the linker may be polyethylene glycol (PEG) represented by Formula 2 below, but is not limited thereto: 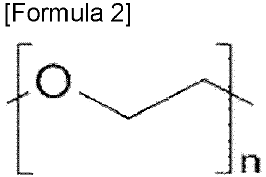 wherein n is 10 to 2,400, n is 10 to 480, or n is 50 to 250, but the range of n is not limited thereto.

In the long-acting conjugate above, the PEG moiety may include not only the -(CH₂CH₂O)ₙ- structure, but also an oxygen atom interposed between a linking element and the -(CH₂CH₂O)ₙ- structure, but the PEG moiety is not limited thereto.

In one embodiment, the ethylene glycol repeating unit may be represented by, for example, [OCH₂CH₂]n, wherein, the value of n is a natural number, and the average molecular weight of the [OCH₂CH₂]n region in the peptide conjugate, *e.g.,* the number average molecular weight, may be determined to be greater than 0 to about 100 kDa, but is not limited thereto. In another example, the value of n is a natural number, and the average molecular weight of the [OCH₂CH₂]n region in the peptide conjugate, *e.g.,* the number average molecular weight, may be about 1 to about 100 kDa, about 1 to about 80 kDa, about 1 to about 50 kDa, about 1 to about 30 kDa, about 1 to about 25 kDa, about 1 to about 20 kDa, about 1 to about 15 kDa, about 1 to about 13 kDa, about 1 to about 11 kDa, about 1 to about 10 kDa, about 1 to about 8 kDa, about 1 to about 5 kDa, about 1 to about 3.4 kDa, about 3 to about 30 kDa, about 3 to about 27 kDa, about 3 to about 25 kDa, about 3 to about 22 kDa, about 3 to about 20 kDa, about 3 to about 18 kDa, about 3 to about 16 kDa, about 3 to about 15 kDa, about 3 to about 13 kDa, about 3 to about 11 kDa, about 3 to about 10 kDa, about 3 to about 8 kDa, about 3 to about 5 kDa, about 3 to about 3.4 kDa, about 8 to about 30 kDa, about 8 to about 27 kDa, about 8 to about 25 kDa, about 8 to about 22 kDa, about 8 to about 20 kDa, about 8 to about 18 kDa, about 8 to about 16 kDa, about 8 to about 15 kDa, about 8 to about 13 kDa, about 8 to about 11 kDa, about 8 to about 10 kDa, about 9 to about 15 kDa, about 9 to about 14 kDa, about 9 to about 13 kDa, about 9 to about 12 kDa, about 9 to about 11 kDa, about 9.5 to about 10.5 kDa, or about 10 kDa, but is not limited thereto.

Additionally, in a specific embodiment, the long-acting conjugate of Chemical 1 above may have a structure in which an immunoglobulin region (F) is linked to the peptide (X) including the amino acid sequences of General Formulae 1 to 3 by a covalent bond through a linker containing an ethylene glycol repeating unit, but the structure of the conjugate is not limited thereto.

The polyethylene glycol is a general term including all of the forms of homopolymers of ethylene glycol, PEG copolymers, or monomethyl-substituted PEG polymers (mPEG), but is not particularly limited thereto.

The molecular weight of the non-peptidyl polymer may be in the range of 0 to about 100 kDa, specifically 1 kDa to 20 kDa, or 1 kDa to 10 kDa, but is not limited thereto. Additionally, the non-peptidyl linker of the present invention, which is linked to the polypeptide corresponding to the F, may include not only a single kind of a polymer but also a combination of different kinds of polymers.

In one embodiment, both ends of the linker may be bound to a thiol group, an amino group, or a hydroxyl group of the immunoglobulin Fc region, and may be bound to a thiol group, an amino group, an azide group, or a hydroxyl group of the peptide (X), but the linker is not limited thereto.

Specifically, the linker may include a reactive group capable of binding to each of the immunoglobulin Fc region and the peptide (X) at both ends. Specifically, the linker may include a reactive group that can bind to a thiol group of cysteine; an amino group located at the N-terminus, lysine, arginine, glutamine, and/or histidine; and/or a hydroxyl group located at the C-terminus of the immunoglobulin Fc region, and that can bind to a thiol group of cysteine; an amino group of lysine, arginine, glutamine, and/or histidine; an azide group of azido-lysine; and/or a hydroxyl group of the peptide (X), but the reactive groups are not limited thereto.

More specifically, the reactive group of the linker may be one or more selected from the group consisting of an aldehyde group, a maleimide group, and a succinimide derivative, but is not limited thereto.

In the above, as an example of the aldehyde group, a propionaldehyde group or butyraldehyde group may be used, but the aldehyde group is not limited thereto.

In the above, as the succinimide derivative, succinimidyl valerate, succinimidyl methylbutanoate, succinimidyl methylpropionate, succinimidyl butanoate, succinimidyl propionate, N-hydroxysuccinimide, hydroxy succinimidyl, succinimidyl carboxymethyl, or succinimidyl carbonate may be used, but the succinimide derivative is not limited thereto.

The linker may be linked to F, the immunoglobulin Fc, and X, the peptide (triple agonist), through the reactive groups to be converted to a linker moiety.

Additionally, the final product produced through reductive amination (or reductive alkylation) by an aldehyde bond is much more stable than that linked by an amide bond. The aldehyde reactive group selectively reacts at the N-terminus at a low pH, while it can form a covalent bond with a lysine residue at a high pH (*e.g.,* pH 9.0), but is not limited thereto.

The reactive groups at both ends of the linker of the present invention may be the same as or different from each other. The linker may have an aldehyde reactive group at both ends. Alternatively, the linker may have an aldehyde group and a maleimide group at each end, or may have an aldehyde group and a succinimide reactive group at each end, but is not limited as long as F, specifically the immunoglobulin Fc region, and X can be bound to each end of the linker.

For example, the linker may have a maleimide group at one end and an aldehyde group, a propionaldehyde group, or a butyraldehyde group at the other end. As another example, the linker may have a succinimidyl group at one end and a propionaldehyde group or a butyraldehyde group at the other end.

When a polyethylene glycol having a hydroxy reactive group at the propionaldehyde end is used as a linker, the conjugate of the present invention may be prepared by activating the hydroxy group to various reactive groups by known chemical reactions or using a commercially available polyethylene glycol having a modified reactive group.

In a specific embodiment, the reactive group of the linker may be linked to a cysteine residue of the peptide (X), more specifically to the -SH group of cysteine, but the linker is not limited thereto.

When maleimide-PEG-aldehyde is used, the maleimide group may be linked to the -SH group of the peptide by a thioether bond, and the aldehyde group may be linked to the -NH₂ group of the immunoglobulin Fc through reductive alkylation, but is not limited thereto, and this is merely an embodiment.

Through the reductive alkylation, a structure such as -PEG-O-CH₂CH₂CH₂NH-immunoglobulin Fc may be formed by linking an amino group at the N-terminus of the immunoglobulin Fc region to an oxygen atom located at one end of the PEG through a linker reactive group having a structure of -CH₂CH₂CH₂-; and a structure, in which one end of the PEG is linked to a sulfur atom located at the cysteine of the peptide through a thioether bond, may be formed. The thioether bond described above may include the following structure:

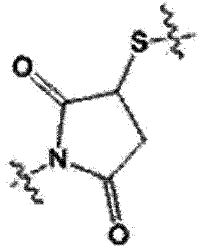

However, the linker is not particularly limited to the above embodiment, and it is merely an embodiment.

Additionally, in the conjugate above, the reactive group of the linker may be linked to -NH₂ located at the N-terminus of the immunoglobulin Fc region, but this is merely an embodiment.

In addition, in the conjugate above, the peptide according to the present invention may be linked to a linker having a reactive group through the C-terminus, but this is merely an embodiment.

As used herein, the term "C-terminus" refers to a carboxy terminus of a peptide, and refers to a position capable of binding with a linker for the purpose of the present invention. For example, the C-terminus may include all of the amino acid residue at the most terminal end of the C-terminus and amino acid residues near the C-terminus, and specifically, may include the 1^{st} to 20^{th} amino acid residues from the most terminal end, although the C-terminus is not limited thereto.

In one embodiment, the conjugate of Formula 1 may have a structure of Formula 3 below:

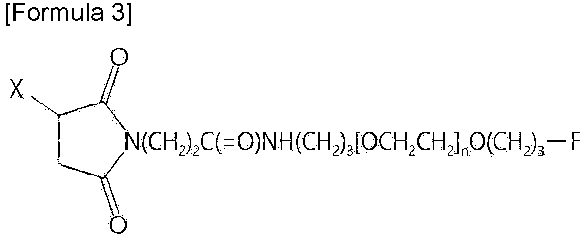

In Formula 3 above, X is the peptide (triple agonist) described above;
F is a human immunoglobulin Fc region; and
n may be a natural number. In particular, the description of n is the same as described above.

In one embodiment, the long-acting conjugate of Formula 3 has a structure in which the peptide X and a human immunoglobulin Fc region F are covalently linked through an ethylene glycol repeating unit, wherein each X may be linked to a succinimide ring of Formula 3, and F may be linked to an oxypropylene group of Formula 3.

In Formula 3 above, the value of n may be determined such that the average molecular weight of the [OCH₂CH₂]n region in the peptide conjugate, *e.g.,* the number average molecular weight, is 1 to 100 kDa, or 1 to 20 kDa, or 10 kDa, but not limited thereto.

In one embodiment, the moiety at which X is linked to the succinimide ring of Formula 3 may be a sulfur atom of the C-terminal cysteine of X.

The moiety linked to the oxypropylene group in F is not specifically limited. In one embodiment of the present invention, the moiety of F linked to the oxypropylene group may be an N-terminal nitrogen or a nitrogen atom of a residue in F (*e.g.,* epsilon nitrogen of lysine). In a specific embodiment of the present invention, the moiety where F is linked to the oxypropylene group may be the N-terminal proline of F, but is not limited thereto.

Additionally, in the conjugate above, the reactive group of the non-peptide polymer may be linked to -NH ₂ located at the N-terminus of the immunoglobulin Fc region, but this is merely an embodiment.

In a specific embodiment, the F in Formula 1 is an immunoglobulin Fc region. For example, the immunoglobulin Fc region may be derived from IgG, but is not particularly limited thereto.

As used herein, the term "immunoglobulin Fc region" refers to a region including the heavy chain constant region 2 (CH2) and/or the heavy chain constant region 3 (CH3), excluding the heavy chain and light chain variable regions of an immunoglobulin. The immunoglobulin Fc region may be one constitution constituting the moiety of the long-acting conjugate of the present invention. The immunoglobulin Fc region may be used interchangeably with "immunoglobulin Fc fragment".

The Fc region in the present specification encompasses not only a native sequence obtained from papain digestion of an immunoglobulin, but also derivatives thereof, for example, variants, in which one or more amino acid residues in the native sequence are converted by deletion, insertion, non-conservative or conservative substitution, or a combination thereof, and thus the sequence become different from the native sequence, *etc.* The derivatives, substituents, and variants above are required to retain FcRn-binding ability. In the present invention, F may be a human immunoglobulin region, but is not limited thereto. The F may have a structure in which two polypeptide chains are linked by an inter-disulfide bond, or a structure in which F is linked through a nitrogen atom in only one of the two chains, but is not limited thereto. The linkage through the nitrogen atom may be linkage to the epsilon amino atom of lysine or the N-terminal amino group by reductive amination.

The reductive amination reaction refers to a reaction in which an amine group or amino group of a reactant reacts with an aldehyde (*i.e.,* a functional group capable of reductive amination) of another reactant to produce an amine, and thereafter, an amine bond is formed by a reduction reaction. The reductive amination reaction is a reaction of organic synthesis widely known in the art.

In one embodiment, the F may be linked through the nitrogen atom of the N-terminal proline thereof, but is not limited thereto.

Such an immunoglobulin Fc region may include a hinge region in the heavy chain constant region, but is not limited thereto.

In the present invention, the immunoglobulin Fc fragment may include a specific hinge sequence in the N-terminus.

As used herein, the term "hinge sequence" refers to a region which is located in the heavy chain and forms a dimer of immunoglobulin Fc regions through an inter-disulfide bond.

In the present invention, the hinge sequence may be modified such that a part of the hinge sequence having the following amino acid sequence is deleted and thus there is only one cysteine residue in the sequence, but is not limited thereto:
Glu-Ser-Lys-Tyr-Gly-Pro-Pro-Cys-Pro-Ser-Cys-Pro (SEQ ID NO: 119).

The hinge sequence may be one in which the 8^{th} or 11^{th} cysteine residue in the hinge sequence of SEQ ID NO: 119 is deleted and thus only one cysteine residue is included in the sequence. The hinge sequence of the present invention may consist of 3 to 12 amino acids, including only one cysteine residue, but the hinge sequence is not limited thereto. More specifically, the hinge sequence of the present invention may have the following sequences: Glu-Ser-Lys-Tyr-Gly-Pro-Pro-Pro-Ser-Cys-Pro(SEQ ID NO: 120), Glu-Ser-Lys-Tyr-Gly-Pro-Pro-Cys-Pro-Ser-Pro(SEQ ID NO: 121), Glu-Ser-Lys-Tyr-Gly-Pro-Pro-Cys-Pro-Ser(SEQ ID NO: 122), Glu-Ser-Lys-Tyr-Gly-Pro-Pro-Cys-Pro-Pro(SEQ ID NO: 123), Lys-Tyr-Gly-Pro-Pro-Cys-Pro-Ser(SEQ ID NO: 124), Glu-Ser-Lys-Tyr-Gly-Pro-Pro-Cys(SEQ ID NO: 125), Glu-Lys-Tyr-Gly-Pro-Pro-Cys(SEQ ID NO: 126), Glu-Ser-Pro-Ser-Cys-Pro(SEQ ID NO: 127), Glu-Pro-Ser-Cys-Pro(SEQ ID NO: 128), Pro-Ser-Cys-Pro(SEQ ID NO: 129), Glu-Ser-Lys-Tyr-Gly-Pro-Pro-Ser-Cys-Pro(SEQ ID NO: 130), Lys-Tyr-Gly-Pro-Pro-Pro-Ser-Cys-Pro(SEQ ID NO: 131), Glu-Ser-Lys-Tyr-Gly-Pro-Ser-Cys-Pro(SEQ ID NO: 132), Glu-Ser-Lys-Tyr-Gly-Pro-Pro-Cys(SEQ ID NO: 133), Lys-Tyr-Gly-Pro-Pro-Cys-Pro(SEQ ID NO: 134), Glu-Ser-Lys-Pro-Ser-Cys-Pro(SEQ ID NO: 135), Glu-Ser-Pro-Ser-Cys-Pro(SEQ ID NO: 136), Glu-Pro-Ser-Cys(SEQ ID NO: 137), Ser-Cys-Pro(SEQ ID NO: 138).

More specifically, the hinge sequence may include the amino acid sequence of SEQ ID NO: 129(Pro-Ser-Cys-Pro) or SEQ ID NO: 138(Ser-Cys-Pro), but is not limited thereto.

The immunoglobulin Fc region of the present invention may be in a form in which two molecules of the immunoglobulin Fc chain form a dimer due to the presence of a hinge sequence therein, and in addition, the conjugate of Formula 1 of the present invention may be in a form in which one end of the linker is linked to one chain of the dimeric immunoglobulin Fc regions, but the immunoglobulin Fc region is not limited thereto.

As used herein, the term "N-terminus" refers to the amino terminus of a protein or polypeptide, and it may include 1, 2, 3, 4, 5, 6, 7, 8, 9, or 10 or more amino acids from the most terminal end or the most terminal end of the amino terminus. The immunoglobulin Fc region of the present invention may include a hinge sequence in the N-terminus, but is not limited thereto.

Additionally, the immunoglobulin Fc region of the present invention may be an extended Fc region, which includes all or part of the heavy chain constant region 1 (CH1) and/or light chain constant region 1 (CL1) excluding the heavy chain and light chain variable regions of an immunoglobulin, as long as it has substantially the same or an improved effect compared to its native type. Additionally, the immunoglobulin Fc region of the present invention may be a region in which some considerably long amino acid sequences corresponding to CH2 and/or CH3 are removed.

For example, the immunoglobulin Fc region of the present invention may be 1) a CH1 domain, a CH2 domain, a CH3 domain, and a CH4 domain; 2) a CH1 domain and a CH2 domain; 3) a CH1 domain and a CH3 domain; 4) a CH2 domain and a CH3 domain; 5) a combination between one or two or more domains among a CH1 domain, a CH2 domain, a CH3 domain and a CH4 domain, and an immunoglobulin hinge region (or part of the hinge region), (e.g., a combination between a CH2 domain and CH3 domain, and a hinge region or part thereof, and a dimeric form of two polypeptides having the above-mentioned combination); and 6) a dimer of each domain of the heavy chain constant region and a light chain constant region, but is not limited thereto.

In the present invention, the immunoglobulin Fc region may be in the form of a dimer or a multimer, composed of single-chain immunoglobulins consisting of domains of the same origin, but is not limited thereto.

Additionally, in one embodiment, the immunoglobulin Fc region may have a dimeric form, and one molecule of X may be covalently linked to one Fc region in a dimeric form, in particular, the immunoglobulin Fc and X may be covalently linked to each other through a non-peptidyl polymer. Meanwhile, it is also possible that two molecules of X are symmetrically conjugated to one Fc region in a dimeric form. In particular, the immunoglobulin Fc and X may be linked to each other through a non-peptidyl polymer, but is not limited to the embodiments described above.

Additionally, the immunoglobulin Fc region of the present invention includes natural amino acid sequences as well as sequence derivatives thereof. The amino acid sequence derivative means that the sequence of the amino acid is different from that of its natural amino acid sequence due to deletion, insertion, non-conservative or conservative substitution, or a combination thereof in one or more amino acid residues in the natural amino acid sequence.

For example, the amino acid residues at positions 214 to 238, 297 to 299, 318 to 322, or 327 to 331 in IgG Fc, which are known to be important for linkage, may be used as the sites suitable for modification.

Additionally, various types of derivatives are possible, for example, one where the site capable of forming an inter-disulfide bond is removed; one where several N-terminal amino acids from native Fc are removed; one where a methionine residue is added to the N-terminus of native Fc, *etc.* Additionally, complement binding sites (e.g., C1q binding sites) or antibody-dependent cell-mediated cytotoxicity (ADCC) sites may be removed to eliminate the effector function. The techniques for preparing the sequence derivatives of these immunoglobulin Fc regions are disclosed in International Publication Nos. WO 97/34631, WO 96/32478, *etc.*

Amino acid substitutions in a protein or peptide that do not alter the entire activity of a molecule are well known in the art (H. Neurath, R. L. Hill, The Proteins, Academic Press, New York, 1979). The most common substitutions occur between amino acid residues of Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, Asp/Gly. In some cases, amino acids may be modified by phosphorylation, sulfation, acrylation, glycosylation, methylation, farnesylation, acetylation, amidation, *etc.*

The Fc derivatives described above may be those which exhibit the same biological activity as the Fc region of the present invention and have an increased structural stability of the Fc region against heat, pH, *etc.*

Additionally, such an Fc fragment may be obtained from a native type isolated from humans or animals (*e.g.,* cows, goats, pigs, mice, rabbits, hamsters, rats, guinea pigs, *etc*.) or may be recombinants or derivatives thereof obtained from transformed animal cells or microorganisms. In particular, the Fc region may be obtained from a native immunoglobulin by isolating a whole immunoglobulin from a living body of human or animal and then treating the isolated immunoglobulin with protease. When the whole immunoglobulin is treated with papain, it is cleaved into Fab and Fc, whereas when treated with pepsin, it is cleaved into pF'c and F(ab)₂. The Fc or pF'c may be isolated by size exclusion chromatography, *etc.* In a more specific embodiment, the Fc region may be a recombinant immunoglobulin Fc region where a human-derived Fc fragment is obtained from a microorganism.

Additionally, the immunoglobulin Fc region may be in the form of native glycans, increased or decreased glycans compared to the native type, or in a deglycosylated form. The increase, decrease, or removal of the immunoglobulin Fc glycans may be achieved by conventional methods such as a chemical method, enzymatic method, and genetic engineering method using a microorganism. In particular, the immunoglobulin Fc region where the glycans are removed from the Fc shows a significant decrease in binding affinity for the complement (C1q) and a decrease or removal of antibody-dependent cytotoxicity or complement-dependent cytotoxicity, and thus it does not induce unnecessary immune responses *in vivo.* In this regard, the immunoglobulin Fc region in a deglycosylated or aglycosylated form may be more suitable to meet the original object of the present invention as a drug carrier.

As used herein, the term "deglycosylation" refers to an Fc region in which glycans are removed with an enzyme, and the term "aglycosylation" refers to a non-glycosylated Fc region produced in prokaryotes, in a more specific embodiment, E. *coli.*

Meanwhile, the immunoglobulin Fc region may be derived from humans or animals (e.g., cows, goats, pigs, mice, rabbits, hamsters, rats, guinea pigs, *etc*.)*,* and in a more specific embodiment, it may be derived from humans.

Additionally, the immunoglobulin Fc region may be derived from IgG, IgA, IgD, IgE, IgM, or a combination or hybrid thereof. In a more specific embodiment, the immunoglobulin Fc region may be derived from IgG or IgM, which are among the most abundant proteins in human blood, and in an even more specific embodiment, it may be derived from IgG, which is known to enhance the half-lives of ligand-binding proteins. In an even yet more specific embodiment, the immunoglobulin Fc region may be an IgG4 Fc fragment, and in the most specific embodiment, it may be an aglycosylated Fc region derived from a human IgG4, but is not limited thereto.

Additionally, in a specific embodiment, the immunoglobulin Fc region, which is a human IgG4 Fc region, may be in the form of a homodimer in which two monomers are linked through an inter-disulfide bond (an inter-chain form) between cysteines, which are the 3^{rd} amino acid of each monomer. In particular, each monomer of the homodimer independently has/or can have an intra-disulfide bond between the cysteines at positions 35 and 95; and an intra-disulfide bond between the cysteines at positions 141 and 199 (i.e., two intra-disulfide bonds (an intra-chain form)). With respect to the number of amino acids, each monomer may consist of 221 amino acids, and the amino acids forming the homodimer may consist of a total of 442 amino acids, but the number of amino acids is not limited thereto. Specifically, the immunoglobulin Fc region may be one in which two monomers having the amino acid sequence of SEQ ID NO: 139 (consisting of 221 amino acids) form a homodimer through an inter-disulfide bond between cysteines, which are the 3^{rd} amino acid of each monomer, and in which the monomers of the homodimer independently form an intra-disulfide bond between the cysteines at positions 35 and 95 and an intra-disulfide bond between the cysteines at positions 141 and 199, but is not limited thereto.

The F in Formula 1 above may include a monomer of the amino acid sequence of SEQ ID NO: 139, and the F may be a homodimer of the monomers of the amino acid sequence of SEQ ID NO: 139, but is not limited thereto.

In one example, the immunoglobulin Fc region may be a homodimer including the amino acid sequence of SEQ ID NO: 140 (consisting of 442 amino acids), but is not limited thereto.

Meanwhile, as used herein, the term "combination" means that polypeptides encoding single-chain immunoglobulin Fc region of the same origin are linked to a single-chain polypeptide of a different origin to form a dimer or multimer. That is, it is possible to prepare a dimer or multimer from two or more fragments selected from the group consisting of Fc fragments of IgG Fc, IgA Fc, IgM Fc, IgD Fc, and IgE Fc.

Additionally, the above-described conjugate may have an increased efficacy of duration compared to native glucagon, native GLP-1 or native GIP, or X, which is not modified with F, and such a conjugate is not only in the above-described form, but also in the form encapsulated in biodegradable nanoparticles, *etc.*

The composition including the liver-targeted drug, for example, the composition including the peptide (e.g., the peptide itself, or in the form in which the peptide is linked to the immunoglobulin Fc region) may be for the prevention or treatment of diseases requiring drug action in the liver.

As used herein, the term "prevention" refers to all activities that inhibit or delay the occurrence of diseases requiring drug action in the liver by administering the composition including the liver-targeted drug, and the term "treatment" refers to all activities that improve or advantageously change the symptoms of diseases requiring drug action in the liver by administering the composition including the liver-targeted drug.

As used herein, the term "administration" refers to the introduction of a particular material into a patient by any appropriate method, and the administration route of the composition is not particularly limited, but may be any conventional route that enables delivery of the composition to the target in the body (*e.g.,* intraperitoneal administration, intravenous administration, intramuscular administration, subcutaneous administration, intradermal administration, oral administration, topical administration, intranasal administration, intrapulmonary administration, or intrarectal administration, *etc*.).

As used herein, the term "disease requiring drug action in the liver" refers to a disease in which the liver-targeted drug according to the present invention can exhibit therapeutic activity, and refers to a disease which can exhibit a preventive or therapeutic effect as the distribution of the drug administered to the liver tissue is increased. For example, any disease that can be targeted by the liver-targeted drug of the present invention to be treated may be included without limitation. Example thereof may include a liver disease. As used herein, the term "liver disease" refers to a disease occurring in the liver, and may include metabolic liver diseases or liver inflammation, but is not limited thereto. Representative examples of the liver disease include simple steatosis, non-alcoholic fatty liver, liver inflammation, non-alcoholic steatohepatitis, cholestatic liver disease, liver fibrosis, cirrhosis, liver failure, and liver cancer, *etc.,* and it may be the liver disease according to the present invention as long as an abnormality occurs in the tissues and functions of the liver. In many cases, liver inflammation may be caused by, viruses, alcohol, drugs, immune disorders, metabolic diseases, *etc.,* and liver inflammation is known to develop into diseases such as cirrhosis, liver cancer, *etc.* according to the progression and chronicity of liver inflammation. The composition according to the present invention may exhibit an effect on liver diseases accompanied by or caused by liver inflammation (*e.g.,* liver inflammation, non-alcoholic steatohepatitis, or liver fibrosis), but the liver disease is not limited thereto. Meanwhile, the composition according to the present invention may also exhibit an effect for preventing or treating liver diseases that do not accompany inflammation, and examples of such liver disease may include simple steatosis, non-alcoholic fatty liver, cirrhosis, *etc.,* but the liver disease is not limited thereto.

The liver disease for which the peptide of the present invention or a conjugate thereof has a therapeutic effect may be a metabolic liver disease, but is not limited thereto. The metabolic liver disease is a disease caused by an abnormal chemical reaction of the body that interferes with the body's metabolism, and may include simple steatosis, fatty liver, steatohepatitis, *etc.*

The composition according to the present invention may be one which exhibits a preventive or therapeutic effect on metabolic liver diseases by reducing the amount of triglycerides and/or cholesterol in liver tissue when administered, but is not limited thereto. The metabolic liver disease may or may not be accompanied by inflammation, and examples of liver diseases that can be treated with the composition according to the present invention include simple steatosis, non-alcoholic fatty liver, non-alcoholic steatohepatitis, *etc.,* but are not limited thereto.

The "nonalcoholic fatty liver disease (NAFLD)", which is a representative example of metabolic liver disease, refers to the case where it is accompanied by fatty liver even though the subject has no history of alcohol intake or is not related to alcohol intake. Fatty liver refers to the occurrence of a phenomenon in which triglycerides appear to be abnormally deposited in liver cells, unlike normal cases. About 5% of the normal liver is composed of adipose tissue, and although triglycerides, fatty acids, phospholipids, cholesterol, and cholesterol esters are the main components of fat, once fatty liver occurs, most of the components are replaced with triglycerides, and when the amount of triglycerides is 5% or higher relative to the liver weight, it is diagnosed as fatty liver. Fatty liver is caused by a disorder of fat metabolism in liver cells or a defect in the process of transporting excessive fat, *etc.,* and it is mainly caused by a disorder of fat metabolism in the liver. Most of the fat accumulated in the fatty liver may be triglycerides.

The non-alcoholic steatohepatitis disease refers to a group of diseases which includes simple steatosis with only excessive accumulation of fat in liver cells, non-alcoholic fatty liver, non-alcoholic steatohepatitis (NASH) accompanied by hepatocellular necrosis, inflammation and fibrosis, *etc.,* but is not limited thereto as long as the disease can be treated with the composition according to the present invention. The non-alcoholic steatohepatitis disease according to the present invention may be non-alcoholic steatohepatitis, but the non-alcoholic steatohepatitis disease is not limited thereto.

Additionally, the liver disease for which the peptide of the present invention or a conjugate thereof has a therapeutic effect may be liver inflammation, but is not limited thereto. As used herein, the term "liver inflammation", which is the most common cause of liver disease, refers to a disease that causes inflammation of the liver, and is divided into acute hepatitis and chronic hepatitis according to causes and symptoms. Viruses, alcohol, drugs, immune disorders, metabolic diseases, *etc.* are the main causes.

As used herein, "non-alcoholic steatohepatitis (NASH)", which is one of the non-alcoholic steatohepatitis diseases, is a representative example of liver disease accompanied by liver cell necrosis, inflammation, and fibrosis. The composition according to the present invention can suppress liver inflammation and fibrosis and thereby exhibits an effect on non-alcoholic steatohepatitis, and specifically, may exhibit an effect on non-alcoholic steatohepatitis accompanied by fatty liver, liver fibrosis, or cirrhosis; or liver cancer caused by non-alcoholic steatohepatitis, but the diseases are not limited thereto.

As used herein, "liver fibrosis" refers to the formation of excessive fibrous connective tissue in organs or tissues during a reparative or responsive process as a result of a wound healing process for repeated liver damage. Chronicity and aggravation of liver inflammation are known to be the cause of the occurrence of liver fibrosis. Liver fibrosis is known to be reversible (unlike cirrhosis), to be composed of thin fibrils, and to have no nodule formation. Once the cause of liver damage ceases, the recovery of normal liver may be possible. However, when the liver fibrosis process is repeated continuously, the crosslinking between the extra cellular matrices (ECMs) increases, thereby resulting in the progression of irreversible cirrhosis with nodules. The composition according to the present invention may exhibit a preventive or therapeutic effect on liver fibrosis, and specifically on liver fibrosis accompanied by non-alcoholic steatohepatitis, but is not limited thereto.

As used herein, "cholestasis" refers to a condition in which the bile flow from the liver to the duodenum is slowed or blocked, and "cholestasis liver disease" means that bile formation in the liver is impaired by conditions such as various diseases, expanded jugular nutrition, or side effects of specific drugs (e.g., some antibiotics). Common signs of cholestasis include fatigue, pruritus (itching), jaundice, and xanthoma (deposition of cholesterol-rich substances in the subcutaneous layer). The effects of cholestasis are extreme and broad, causing exacerbation of liver disease to a systemic disease, liver decompensation, and the need for liver transplantation. Causes of cholestasis liver disease may include acute hepatitis, inflammation of the bile ducts, *etc.*

The cholestasis liver disease may include primary biliary cholangitis (PBC), primary sclerosing cholangitis (PSC), progressive familial intrahepatic cholestasis (PFIC), and Alagille syndrome (AS), *etc.,* but is not limited thereto.

Primary biliary cirrhosis, which is also known as primary biliary cholangitis (PBC), is a cryptogenic chronic cholestasis liver disease. Progressive bile duct damage due to portal and periportal inflammation can cause progressive fibrosis and ultimate cirrhosis. Thus far, immunological, genetic, and environmental factors are known as potential causes of primary biliary cirrhosis. Primary biliary cirrhosis mostly occurs in middle-aged women, and symptoms such as fatigue, itching, or unidentified hyperlipidemia may also appear in the early onset of primary biliary cirrhosis.

Primary sclerosing cholangitis (PSC) is a chronic progressive cholestasis liver disease caused by cryptogenic inflammation and fibrosis of the intrahepatic and extrahepatic bile ducts. Specifically, it is an inflammatory disease of the bile ducts and biliary tract, and once the disease progresses, fibrosis occurs and the bile duct wall becomes thickened, thereby narrowing the bile ducts. The causes of the disease have not yet been identified, but it appears that a combination of various factors such as genetic factors, environmental factors, related immune responses, *etc.* may be a possible cause.

As used herein, the "liver cirrhosis" is a chronic disease that occurs with repeated increasing of the regeneration of liver cells and fibrous tissue, it is pathologically accompanied by necrosis, inflammation, and fibrosis, and it ultimately progresses into cirrhosis complications (e.g., liver decompensation) and diseases (e.g., liver cancer), eventually leading to death. In particular, since liver cirrhosis can be discovered only after considerable progress due to the absence of awareness of one's own symptoms in the early stages of the disease, it is required that liver fibrosis, which is a condition before it evolves into cirrhosis, *etc.,* be treated promptly. The composition according to the present invention may exhibit a preventive or therapeutic effect on liver cirrhosis, and specifically on liver cirrhosis accompanied by non-alcoholic steatohepatitis, but is not limited thereto.

As used herein, the "liver decompensation" refers to a condition in which liver function is weakened and the liver cannot perform protein synthesis and metabolic functions as normal physiological functions due to viral hepatitis, cirrhosis, liver damage by drugs or alcohol, or liver disease. Liver decompensation is divided into acute liver decompensation and chronic liver decompensation according to the progression rate, and it is known to cause various complications. Since the composition according to the present invention exhibits effects such as inhibition of inflammation and fibrosis, *etc.,* it may exhibit a preventive or therapeutic effect on liver decompensation.

As used herein, the "hepatocellular carcinoma" refers to a malignant tumor originating from liver cells, and it can be classified as primary liver cancer (hepatocellular carcinoma), which occurs in the liver cells themselves, and metastatic liver cancer, in which cancers of other tissues have metastasized to the liver, and about 90% or more of liver cancer is primary liver cancer. Major causes are alcohol, smoking, obesity, *etc.,* in addition to hepatitis and chronic liver disease. The composition according to the present invention may exhibit a preventive or therapeutic effect on liver cancer, and specifically liver cancer caused by non-alcoholic steatohepatitis, but is not limited thereto.

As disclosed in International Publication No. WO 2020/263063, the triple agonist of the present invention or a long-acting conjugate thereof having excellent effects on liver diseases, *e.g.,* metabolic liver disease, simple steatosis, non-alcoholic fatty liver, liver inflammation, non-alcoholic steatohepatitis, cholestatic liver disease, liver fibrosis, cirrhosis, liver failure and liver cancer, *etc.,* also exhibits excellent effect of targeting to the liver tissue and thereby has excellent preventive or therapeutic effects on the above liver diseases, and thus can be effectively provided as a therapeutic agent for liver diseases.

The pharmaceutical composition of the present invention may further include a pharmaceutically acceptable carrier, excipient, or diluent. The pharmaceutically acceptable carrier, excipient, or diluent may be one that is not naturally occurring.

As used herein, the term "pharmaceutically acceptable" refers to the properties of having a sufficient amount to exhibit a therapeutic effect and not causing adverse effects, and may be easily determined by a skilled person in the art based on the factors well known in the medical field, such as the kind of disease, age, body weight, health status, sex, drug sensitivity of a patient, administration route, administration method, administration frequency, duration of treatment, a drug to be mixed or administered simultaneously in combination, *etc.*

The pharmaceutical composition including the peptide of the present invention may further contain a pharmaceutically acceptable carrier. The pharmaceutically acceptable carrier may include, for oral administration, a binder, a lubricant, a disintegrant, a solubilizing agent, a dispersant, a stabilizing agent, a suspending agent, a coloring agent, a fragrance, *etc*.; for injections, a buffering agent, a preservative, an analgesic, a solubilizing agent, an isotonic agent, a stabilizing agent, *etc.,* which may be combined to be used; and for topical administrations, a base, an excipient, a lubricant, a preservative, *etc.,* although it is not particularly limited thereto.

The formulation type of the composition of the present invention may be prepared variously by combining with a pharmaceutically acceptable carrier described above. For example, for oral administration, the composition may be formulated into tablets, troches, capsules, elixirs, suspensions, syrups, wafers, *etc.,* and for injections, the composition may be formulated into unit-dose ampoules or multi-dose containers. The composition may also be formulated into solutions, suspensions, tablets, pills, capsules, sustained-release formulations, *etc.*

Meanwhile, examples of suitable carriers, excipients, and diluents for formulation may include lactose, dextrose, sucrose, sorbitol, mannitol, xylitol, erythritol, maltitol, starch, acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, *etc.* Additionally, the composition may further include a filler, an anti-coagulant, a lubricant, a humectant, a fragrance, a preservative, *etc.*

Additionally, the pharmaceutical composition of the present invention may have any one formulation type selected from the group consisting of tablets, pills, powders, granules, capsules, suspensions, liquid medicine for internal use, emulsions, syrups, sterile aqueous solutions, non-aqueous solvents, lyophilized formulations, and suppositories.

Additionally, the composition may be formulated into a preparation of a unit dosage form suitable for the administration into a patient's body, and may specifically be formulated into a preparation useful for the administration of protein drugs according to the conventional method in the pharmaceutical field so as to be administered by an oral or parenteral route (including skin, intravenous, intramuscular, intraarterial, intramedullary, intrathecal, intraventricular, pulmonary, transdermal, subcutaneous, intraperitoneal, intranasal, intragastrical, topical, sublingual, vaginal, or rectal route), but is not limited thereto.

Additionally, the conjugate may be used by being mixed with various pharmaceutically acceptable carriers such as physiological saline or organic solvents. To increase stability or absorptivity, carbohydrates (*e.g.,* glucose, sucrose, or dextrans), antioxidants (*e.g.,* ascorbic acid or glutathione), chelating agents, low-molecular weight proteins, or other stabilizers, *etc.* may be used as pharmaceutical drugs.

The administration dose and frequency of the pharmaceutical composition of the present invention are determined by the type of drugs, which are active ingredients, together with various factors, such as the disease to be treated, administration route, patient's age, sex, and body weight, severity of the disease, *etc.*

The total effective dose of the composition of the present invention may be administered to a patient in a single dose or may be administered for a long period of time in multiple doses according to a fractionated treatment protocol. In the pharmaceutical composition of the present invention, the content of active ingredient(s) may vary depending on the severity of the disease. Specifically, the total daily dose of the conjugate of the present invention may be about 0.0001 mg to 500 mg per 1 kg of the body weight of a patient. However, the effective dose of the conjugate is determined considering various factors including patient's age, body weight, health conditions, sex, disease severity, diet, and excretion rate, as well as administration route and treatment frequency of the pharmaceutical composition. In this respect, those skilled in the art may easily determine the effective dose suitable for a particular use of the composition of the present invention. The pharmaceutical composition according to the present invention is not particularly limited to the formulation type and administration route and mode, as long as it shows the effects of the present invention.

The pharmaceutical composition of the present invention may have excellent in *vivo* duration of efficacy and titer, and thus, the number and frequency of administration of the pharmaceutical preparation of the present invention may be significantly reduced.

Another aspect for implementing the present invention provides a physiologically active substance targeted to the liver tissue, specifically, a physiologically active substance targeted to the liver tissue including a substance having a binding affinity to a glucagon receptor, e.g., a liver-targeted drug.

The physiologically active substance targeted to the liver tissue, substance having a binding affinity to a glucagon receptor, liver-targeted drug, *etc.,* are as described above.

Still another aspect for implementing the present invention provides a method for preventing or treating diseases requiring drug acting in the liver, including administering the pharmaceutical composition or the physiologically active substance targeted to the liver tissue to a subject in need thereof.

The pharmaceutical composition, physiologically active substance targeted to the liver tissue, diseases requiring drug acting in the liver, prevention and treatment are as described above.

As used herein, the subject refers to a subject in need of drug action in the liver, *e.g.,* a subject suspected of having a liver disease, and the subject suspected of having a liver disease refers to mammals including humans, rats, livestock, *etc.,* which have or are at risk of developing the liver disease, but any subject which can be treated with the composition including the liver-targeted drug of the present invention is included without limitation.

The method of the present invention may include administering the pharmaceutical composition including the liver-targeted drug in a pharmaceutically effective dose. An appropriate total daily dose of the pharmaceutical composition may be determined within the scope of correct medical judgment by a practitioner, and the pharmaceutical composition may be administered once or several times in divided doses. However, for the purpose of the present invention, it is preferred that the specific therapeutically effective dose of the pharmaceutical composition for any particular patient be applied differently depending on the kind and degree of responses to be achieved, specific compositions including whether other agents are occasionally used therewith, the patient's age, body weight, health conditions, sex and diet, administration time, administration route, excretion rate of the composition, duration of treatment, various factors including drugs used in combination or simultaneously with the specific compositions, and similar factors well known in the medical field.

Yet another aspect for implementing the present invention provides the use of the liver-targeted drug, specifically, a physiologically active substance targeted to the liver, for use in the preparation of a medicament for preventing or treating diseases requiring drug action in the liver.

The liver disease, liver-targeted drug, and physiologically active substance targeted to the liver are as described above.

Even another aspect for implementing the present invention provides a method for inducing the targeting of the liver-targeted drug to the liver tissue by administering the liver-targeted drug, specifically, a physiologically active substance targeted to the liver, to a subject in need thereof.

The liver-targeted drug, physiologically active substance targeted to the liver, and subject are as described above.

Further another aspect for implementing the present invention provides a method, in which the liver-targeted drug, specifically a physiologically active substance targeted to the liver tissue, is administered to a subject in need thereof, thereby inducing an increase in distribution of the physiologically active substance in the liver tissue.

The liver-targeted drug, physiologically active substance targeted to the liver, and subject are as described above.

The method for inducing targeting to the liver may be achieved by an appropriate administration route such that the substance can be targeted to the liver, e.g., by a subcutaneous route, but is not particularly limited thereto.

Hereinafter, the present invention will be described in more detail with reference to the following Examples. However, these Examples are for illustrative purposes only and the scope of the disclosure is not limited by these Examples.

### Example 1: Preparation of Triple Agonists

Triple agonists showing activities to all of glucagon, GLP-1, and GIP receptors were prepared and their amino acid sequences are shown in Table 1 below.

**[Table 1]**

| **SEQ ID NO:** | **Sequence** | **Informat ion** |
|---|---|---|
| 1 | H X Q G T F T S D V S S Y L D G Q A A K E F I A W L V K G C | |
| 2 | H X Q G T F T S D V S S Y L D G Q A Q K E F I A W L V K G C | |
| 3 | H X Q G T F T S D V S S Y L L G Q A A K Q F I A W L V K G G G P S S G A P P P S C | |
| 4 | H X Q G T F T S D V S S Y L L G Q Q Q K E F I A W L V K G C | |
| 5 | H X Q G T F T S D V S S Y L L G Q Q Q K E F I A W L V K G G G P S S G A P P P S C | |
| 6 | H X Q G T F T S D V S S Y L D G Q A A K E F V A W L L K G C | |
| 7 | H X Q G T F T S D V S K Y L D G Q A A K E F V A W L L K G C | |
| 8 | H X Q G T F T S D V S K Y L D G Q A A Q E F V A W L L K G C | |
| 9 | H X Q G T F T S D V S K Y L D G Q A A Q E F V A W L L A G C | |
| 10 | H X Q G T F T S D V S K Y L D G Q A A Q E F V A W L L A G G G P S S G A P P P S C | |
| 11 | CA G E G T F T S D L S K Y L D S R R Q Q L F V Q W L K A G G P S S G A P P P S H G | |
| 12 | CA G E G T F I S D L S K Y M D E Q A V Q L F V E W L M A G G P S S G A P P P S H G | |
| 13 | CA G E G T F I S D Y S I Q L D E I A V Q D F V E W L L A Q K P S S G A P P P S H G | |
| 14 | CA G Q G T F T S D Y S I Q L D E I A V R D F V E W L K N G G P S S G A P P P S H G | |
| 15 | CA G Q G T F T S D L S K Q M D E E A V R L F I E W L K N G G P S S G A P P P S H G | |
| 16 | CA G Q G T F T S D L S K Q M D S E A Q Q L F I E W L K N G G P S S G A P P P S H G | |
| 17 | CA G Q G T F T S D L S K Q M D E E R A R E F I E W L L A Q K P S S G A P P P S H G | |
| 18 | CA G Q G T F T S D L S K Q M D S E R A R E F I E W L K N T G P S S G A P P P S H G | |
| 19 | CA G Q G T F T S D L S I Q Y D S E H Q R D F I E W L K D T G P S S G A P P P S H G | |
| 20 | CA G Q G T F T S D L S I Q Y E E E A Q Q D F V E W L K D T G P S S G A P P P S H G | |
| 21 | Y X Q G T F T S D Y S K Y L D E C R A K E F V Q W L L D H H P S S G Q P P P S | Ring formation |
| 22 | Y X Q G T F T S D Y S K C L D E K R A K E F V Q W L L D H H P S S G Q P P P S | Ring formation |
| 23 | Y X Q G T F T S D Y S K Y L D E C R A K E F V Q W L L A Q K G K K N D W K H N I T | Ring formation |
| 24 | Y X Q G T F T S D Y S K Y L D E C R A K E F V Q W L K N G G P S S G A P P P S | Ring formation |
| 25 | H X Q G T F T S D C S K Y L D E R A A Q D F V Q W L L D G G P S S G A P P P S | |
| 26 | H X Q G T F T S D C S K Y L D S R A A Q D F V Q W L L D G G P S S G A P P P S | |
| 27 | H X Q G T F T S D Y S K Y L D E R A C Q D F V Q W L L D Q G G P S S G A P P P S | |
| 28 | H X Q G T F T S D Y S K Y L D E K R A Q E F V C W L L A Q K G K K N D W K H N I T | |
| 29 | H X Q G T F T S D Y S K Y L D E K A A K E F V Q W L L N T C | Ring formation |
| 30 | H X Q G T F T S D Y S K Y L D E K A Q K E F V Q W L L D T C | Ring formation |
| 31 | H X Q G T F T S D Y S K Y L D E K A C K E F V Q W L L A Q | Ring formation |
| 32 | H X Q G T F T S D Y S K Y L D E K A C K D F V Q W L L D G G P S S G A P P P S | Ring formation |
| 33 | H X Q G T F T S D Y S I A M D E I H Q K D F V N W L L A Q K C | Ring formation |
| 34 | H X Q G T F T S D Y S K Y L D E K R Q K E F V N W L L A Q K C | Ring formation |
| 35 | H X Q G T F T S D Y S I A M D E I H Q K D F V N W L L N T K C | Ring formation |
| 36 | H X Q G T F T S D Y S K Y L C E K R Q K E F V Q W L L N G G P S S G A P P P S G | Ring formation |
| 37 | H X Q G T F T S D Y S K Y L D E C R Q K E F V Q W L L N G G P S S G A P P P S G | Ring formation |
| 38 | CA X Q G T F T S D K S S Y L D E R A A Q D F V Q W L L D G G P S S G A P P P S S | |
| 39 | H X Q G T F T S D Y S K Y L D G Q H A Q C F V A W L L A G G G P S S G A P P P S | |
| 40 | H X Q G T F T S D K S K Y L D E R A C Q D F V Q W L L D G G P S S G A P P P S | |
| 41 | H X Q G T F T S D K S K Y L D E C A A Q D F V Q W L L D G G P S S G A P P P S | |
| 42 | Y X Q G T F T S D Y S K Y L D E K R A K E F V Q W L L D H H P S S G Q P P P S C | Ring formation |
| 43 | Y X Q G T F T S D Y S K Y L D E K R A K E F V Q W L L D H H C S S G Q P P P S | Ring formation |
| 44 | H G Q G T F T S D C S K Q L D G Q A A Q E F V A W L L A G G P S S G A P P P S | |
| 45 | H G Q G T F T S D C S K Y M D G Q A A Q D F V A W L L A G G P S S G A P P P S | |
| 46 | H G Q G T F T S D C S K Y L D E Q H A Q E F V A W L L A G G P S S G A P P P S | |
| 47 | H G Q G T F T S D C S K Y L D G Q R A Q E F V A W L L A G G P S S G A P P P S | |
| 48 | H G Q G T F T S D C S K Y L D G Q R A Q D F V N W L L A G G P S S G A P P P S | |
| 49 | C A X Q G T F T S D Y S IC M D E I H Q K D F V N W L L NT K | Ring formation |
| 50 | H X Q G T F T S D Y S K Y L D E K R A K E F V Q W L L D H H P [?] S S G Q P P P S C | Ring formation |
| 51 | H X Q G T F T S D Y S K Y L D E K R Q K E F V Q W L L N T C | Ring formation |
| 52 | H X Q G T F T S D Y S K Y L D E K R Q K E F V Q W L L D T C | Ring formation |
| 53 | H X E G T F T S D Y S I A M D E I H Q K D F V N W L L A Q C | Ring formation |
| 54 | H X E G T F T S D Y S I A M D E I H Q K D F V D W L L A E C | Ring formation |
| 55 | H X Q G T F T S D Y S I A M D E I H Q K D F V N W L L A Q C | Ring formation |
| 56 | H X Q G T F T S D Y S K Y L D E K R Q K E F V N W L L A Q C | Ring formation |
| 57 | H X Q G T F T S D Y S I A M D E I H Q K D F V N W L L N T C | Ring formation |
| 58 | H X Q G T F T S D Y S K Y L D E K R Q K E F V Q W L L N T K C | Ring formation |
| 59 | C A X Q G T F T S D Y S I C M D E K H Q K D F V N W L L N T K | Ring formation |
| 60 | C A X Q G T F T S D Y S I A M D E K H C K D F V N W L L N T K | Ring formation |
| 61 | C A X Q G T F T S D Y S I A M D E I A C K D F V N W L L N T K | Ring formation |
| 62 | CA X Q G T F T S D K S K Y L D E R A A Q D F V Q W L L D G G P S S G A P P P S | |
| 63 | CA X Q G T F T S D C S K Y L D E R A A Q D F V Q W L L D G G P S S G A P P P S | |
| 64 | Y X Q G T F T S D Y S K Y L D E C A A K E F V Q W L L D H H P S S G Q P P P S | Ring formation |
| 65 | H X Q G T F T S D Y S K C L D E K R A K E F V Q W L L D H H P S S G Q P P P S | Ring formation |
| 66 | Y X Q G T F T S D Y S K Y L D E C R A K D F V Q W L L D H H P S S G Q P P P S | Ring formation |
| 67 | Y X Q G T F T S D Y S K Y L D E C A A K D F V Q W L L D H H P S S G Q P P P S | Ring formation |
| 68 | Y X Q G T F T S D Y S K C L D E K A A K E F V Q W L L D H H P S S G Q P P P S | Ring formation |
| 69 | Y X Q G T F T S D Y S K C L D E R A A K E F V Q W L L D H H P S S G Q P P P S | Ring formation |
| 70 | Y X Q G T F T S D Y S K C L D E K R A K D F V Q W L L D H H P S S G Q P P P S | Ring formation |
| 71 | Y X Q G T F T S D Y S K Y L D E R A C K D F V Q W L L D H H P S S G Q P P P S | Ring formation |
| 72 | Y X Q G T F T S D C S K Y L D E R A A K D F V Q W L L D H H P S S G Q P P P S | Ring formation |
| 73 | CA X Q G T F T S D Y S K Y L D E C R A K E F V Q W L L D H H P S S G Q P P P S | Ring formation |
| 74 | CA X Q G T F T S D Y S K C L D E K R A K E F V Q W L L D H H P S S G Q P P P S | Ring formation |
| 75 | Y X Q G T F T S D Y S K Y L D E K A A K E F V Q W L L D H H P S S G Q P P P S C | Ring formation |
| 76 | Y X Q G T F T S D Y S K Y L D E K R A K D F V Q W L L D H H P S S G Q P P P S C | Ring formation |
| 77 | Y X Q G T F T S D Y S K Y L D E K A A K D F V Q W L L D H H P S S G Q P P P S C | Ring formation |
| 78 | H X Q G T F T S D Y S K Y L D E K R Q K E F V Q W L L D T K C | Ring formation |
| 79 | H X E G T F T S D Y S I A M D E I H Q K D F V N W L L A Q K C | Ring formation |
| 80 | H X E G T F T S D Y S I A M D E I H Q K D F V D W L L A E K C | Ring formation |
| 81 | C A X Q G T F T S D Y S K Y L D E K R Q K E F V Q W L L N T C | Ring formation |
| 82 | C A X Q G T F T S D Y S K Y L D E K R Q K E F V Q W L L D T C | Ring formation |
| 83 | C A X E G T F T S D Y S I A M D E I H Q K D F V N W L L A Q C | Ring formation |
| 84 | C A X E G T F T S D Y S I A M D E I H Q K D F V D W L L A E C | Ring formation |
| 85 | C A X Q G T F T S D Y S I A M D E I H Q K D F V N W L L A Q C | Ring formation |
| 86 | C A X Q G T F T S D Y S K Y L D E K R Q K E F V N W L L A Q C | Ring formation |
| 87 | C A X Q G T F T S D Y S I A M D E I H Q K D F V N W L L N T C | Ring formation |
| 88 | C A X Q G T F T S D Y S K Y L D E K R Q K E F V Q W L L N T K C | Ring formation |
| 89 | C A X Q G T F T S D Y S K Y L D E K R Q K E F V Q W L L D T K C | Ring formation |
| 90 | C A X E G T F T S D Y S I A M D E I H Q K D F V N W L L A Q K C | Ring formation |
| 91 | C A X E G T F T S D Y S I A M D E I H Q K D F V D W L L A E K C | Ring formation |
| 92 | C A X Q G T F T S D Y S I A M D E I H Q K D F V N W L L A Q K C | Ring formation |
| 93 | C A X Q G T F T S D Y S K Y L D E K R Q K E F V N W L L A Q K C | Ring formation |
| 94 | C A X Q G T F T S D Y S I A M D E I H Q K D F V N W L L N T K C | Ring formation |
| 95 | Y X Q G T F T S D Y S K Y L D E K R A K E F V Q W L L C H H P S S G Q P P P S | Ring formation |
| 96 | Y X Q G T F T S D Y S K Y L D E K R A K E F V Q W L L D H C P S S G Q P P P S | Ring formation |
| 97 | Y X Q G T F T S D Y S K Y L D E K R A K E F V Q W L L D C H P S S G Q P P P S | Ring formation |
| 98 | Y X Q G T F T S D Y S K A L D E K A A K E F V N W L L D H H P S S G Q P P P S C | Ring formation |
| 99 | Y X Q G T F T S D Y S K A L D E K A A K D F V N W L L D H H P S S G Q P P P S C | Ring formation |
| 100 | Y X Q G T F T S D Y S K A L D E K A A K E F V Q W L L D Q H P S S G Q P P P S C | Ring formation |
| 101 | Y X Q G T F T S D Y S K A L D E K A A K E F V N W L L D Q H P S S G Q P P P S C | Ring formation |
| 102 | Y X Q G T F T S D Y S K A L D E K A A K D F V N W L L D Q H P S S G Q P P P S C | Ring formation |

In the sequences described in Table 1, the amino acid represented by X represents aminoisobutyric acid (Aib), which is a non-natural amino acid, and the underlined amino acids represent the formation of a ring between the underlined amino acids. Additionally, in Table 1, CA represents 4-imidazoacetyl and Y represents tyrosine. As the triple agonist peptide, a triple agonist having an amidated C-terminus is used if necessary. Meanwhile, the peptide was prepared by a solid-phase peptide synthesis method using a synthesizer, and in case of the triple agonist having an amidated C-terminus, an amide resin was used for amidation of the C-terminus when prepared by a solid-phase peptide synthesis method using a synthesizer.

### Example 2: Preparation of Long-Acting Conjugate of Triple Agonists

For the PEGylation of the cysteine residue of triple agonists (SEQ ID NOS: 21, 22, 42, 43, 50, 77, and 96) of Example 1 using PEG (10 kDa) having a maleimide group and an aldehyde group at each ends, i.e., maleimide-PEG-aldehyde (10 kDa, NOF, Japan), the triple agonists and the maleimide-PEG-aldehyde were reacted at a molar ratio of 1:1 to 3, at a protein concentration of 1 mg/mL to 5 mg/mL at low temperature for 0.5 to 3 hours. In particular, the reaction was performed in an environment in which 20% to 60% isopropanol was added to 50 mM Tris buffer (pH 7.5). Upon completion of the reaction, the reactants were applied to SP sepharose HP (GE Healthcare, USA) to purify the triple agonists which were mono-PEGylated on cysteine.

Then, the purified mono-PEGylated triple agonists and an immunoglobulin Fc (homodimer of SEQ ID NO: 139) were reacted at a molar ratio of 1:1 to 5, at a protein concentration of 10 mg/mL to 50 mg/mL at 4°C to 8°C for 12 to 18 hours. The reaction was performed in an environment in which 10 mM to 50 mM sodium cyanoborohydride (a reducing agent) and 10% to 30% isopropanol were added to 100 mM potassium phosphate butter (pH 6.0). Upon completion of the reaction, the reactants were applied to the Butyl sepharose FF purification column (GE Healthcare, USA) and Source ISO purification column (GE Healthcare, USA) to purify the conjugates including the triple agonists and the immunoglobulin Fc. The purified long-acting conjugates had a structure in which the triple agonist peptide, polyethylene glycol (PEG) linker, and Fc dimer were covalently linked at a molar ratio of 1:1:1 in the molecule, wherein the PEG linker was linked to only one of the two polypeptide chains of the Fc dimer.

Meanwhile, in the immunoglobulin Fc, two monomers having the amino acid sequence of SEQ ID NO: 139 (consisting of 221 amino acids) formed a homodimer through an inter-disulfide bond between cysteines, which were the 3^{rd} amino acid of each monomer, and the monomers of the homodimer independently formed an intra-disulfide bond between the cysteines at positions 35 and 95 and an intra-disulfide bond between the cysteines at positions 141 and 199.

After the preparation, the purity analyzed by reverse phase chromatography, size exclusion chromatography, and ion exchange chromatography was shown to be 95% or higher.

In particular, the conjugate in which the triple agonist of SEQ ID NO: 21 having an amidated C-terminus and the immunoglobulin Fc were linked through PEG was named a 'conjugate including the triple agonist of SEQ ID NO: 21 and the immunoglobulin Fc' or `long-acting conjugate of SEQ ID NO: 21', and these may be used interchangeably in the present invention.

In particular, the conjugate in which the triple agonist of SEQ ID NO: 22 having an amidated C-terminus and the immunoglobulin Fc were linked through PEG was named a 'conjugate including the triple agonist of SEQ ID NO: 22 and the immunoglobulin Fc' or `long-acting conjugate of SEQ ID NO: 22', and these may be used interchangeably in the present invention.

In particular, the conjugate in which the triple agonist of SEQ ID NO: 42 having an amidated C-terminus and the immunoglobulin Fc were linked through PEG was named a 'conjugate including the triple agonist of SEQ ID NO: 42 and the immunoglobulin Fc' or `long-acting conjugate of SEQ ID NO: 42', and these may be used interchangeably in the present invention.

In particular, the conjugate in which the triple agonist of SEQ ID NO: 43 having an amidated C-terminus and the immunoglobulin Fc were linked through PEG was named a 'conjugate including the triple agonist of SEQ ID NO: 43 and the immunoglobulin Fc' or `long-acting conjugate of SEQ ID NO: 43', and these may be used interchangeably in the present invention.

In particular, the conjugate in which the triple agonist of SEQ ID NO: 50 having an amidated C-terminus and the immunoglobulin Fc were linked through PEG was named a 'conjugate including the triple agonist of SEQ ID NO: 50 and the immunoglobulin Fc' or `long-acting conjugate of SEQ ID NO: 50', and these may be used interchangeably in the present invention.

In particular, the conjugate in which the triple agonist of SEQ ID NO: 77 having an amidated C-terminus and the immunoglobulin Fc were linked through PEG was named a 'conjugate including the triple agonist of SEQ ID NO: 77 and the immunoglobulin Fc' or `long-acting conjugate of SEQ ID NO: 77', and these may be used interchangeably in the present invention.

In particular, the conjugate in which the triple agonist of SEQ ID NO: 96 having an amidated C-terminus and the immunoglobulin Fc were linked through PEG was named a 'conjugate including the triple agonist of SEQ ID NO: 96 and the immunoglobulin Fc' or `long-acting conjugate of SEQ ID NO: 96', and these may be used interchangeably in the present invention.

### Experimental Example 1: Measurement of in vitro Activities of Triple Agonists and Long-Acting Conjugates Thereof

In order to measure the activities of the triple agonists and the long-acting conjugates thereof prepared in Examples 1 and 2, the cell activity was measured *in vitro* using cell lines in which the GLP-1 receptor, glucagon (GCG) receptor, and GIP receptor were transformed, respectively.

Each of the cell lines above is one in which the genes for the human GLP-1 receptor, human GCG receptor, and human GIP receptor were transformed in Chinese hamster ovary (CHO), respectively, to be expressed therein, and is thus suitable for the measurement of the activities of GLP-1, GCG, and GIP. Accordingly, the activity for each part was measured using the respective transformed cell line.

For the measurement of the GLP-1 activity of the triple agonists and long-acting conjugates thereof prepared in Examples 1 and 2, human GLP-1 was subjected to a 4-fold serial dilution from 50 nM to 0.000048 nM, and the triple agonists and long-acting conjugates thereof prepared in Examples 1 and 2 were subjected to a 4-fold serial dilution from 400 nM to 0.00038 nM. The culture solution was removed from the cultured CHO cells, in which the human GLP-1 receptor was expressed, and each of the serially diluted materials was added to the CHO cells in an amount of 5 µL, respectively, and a buffer solution containing a cAMP antibody was added thereto in an amount of 5 µL and cultured at room temperature for 15 minutes. Then, a detection mix containing a cell lysis buffer was added thereto in an amount of 10 µL for the lysis of the cells and reacted at room temperature for 90 minutes. The cell lysates, after completion of the reaction, were applied to the LANCE cAMP kit (PerkinElmer, USA) to calculate the EC₅₀ value through accumulated cAMP, and the values were compared with one another. The relative titers compared to human GLP-1 are shown in Tables 2 and 3 below.

For the measurement of the GCG activity of the triple agonists and long-acting conjugates thereof prepared in Examples 1 and 2, human GCG was subjected to a 4-fold serial dilution from 50 nM to 0.000048 nM, and the triple agonists and long-acting conjugates thereof prepared in Examples 1 and 2 were subjected to a 4-fold serial dilution from 400 nM to 0.00038 nM. The culture solution was removed from the cultured CHO cells, in which the human GCG receptor was expressed, and each of the serially diluted materials was added to the CHO cells in an amount of 5 µL, respectively, and a buffer solution containing a cAMP antibody was added thereto in an amount of 5 µL and cultured at room temperature for 15 minutes. Then, a detection mix containing a cell lysis buffer was added thereto in an amount of 10 µL for the lysis of the cells and reacted at room temperature for 90 minutes. The cell lysates, after completion of the reaction, were applied to the LANCE cAMP kit (PerkinElmer, USA) to calculate the EC₅₀ value through accumulated cAMP, and the values were compared with one another. The relative titers compared to human GCG are shown in Tables 2 and 3 below.

For the measurement of the GIP activity of the triple agonists and long-acting conjugates thereof prepared in Examples 1 and 2, human GIP was subjected to a 4-fold serial dilution from 50 nM to 0.000048 nM, and the triple agonists and long-acting conjugates thereof prepared in Examples 1-1 and 1-2 were subjected to a 4-fold serial dilution from 400 nM to 0.00038 nM. The culture solution was removed from the cultured CHO cells, in which the human GIP receptor was expressed, and each of the serially diluted materials was added to the CHO cells in an amount of 5 µL, respectively, and a buffer solution containing a cAMP antibody was added thereto in an amount of 5 µL and cultured at room temperature for 15 minutes. Then, a detection mix containing a cell lysis buffer was added thereto in an amount of 10 µL for the lysis of the cells and reacted at room temperature for 90 minutes. The cell lysates, after completion of the reaction, were applied to the LANCE cAMP kit (PerkinElmer, USA) to calculate the EC₅₀ value through accumulated cAMP, and the values were compared with one another. The relative titers compared to human GIP are shown in Tables 2 and 3 below. The triple agonist peptides of SEQ ID NOS: 1 to 102 whose activities were confirmed in Table 2 below and the triple agonist peptides in the long-acting conjugates of the triple agonists whose activities were confirmed in Table 3 are triple agonists having C-terminus that is amidated.

**[Table 2]**

| Relative Titer Ratio of Triple Agonists | | | |
|---|---|---|---|
| | *in vitro* Activity Compared to Native Peptide (%) | | |
| **SEQ ID NO:** | vs GLP-1 | vs Glucagon | vs GIP |
| 1 | 3.2 | <0.1 | <0.1 |
| 2 | 5.9 | <0.1 | <0.1 |
| 3 | 1.8 | <0.1 | <0.1 |
| 4 | 8.5 | <0.1 | <0.1 |
| 5 | 42.1 | <0.1 | <0.1 |
| 6 | 17.0 | <0.1 | <0.1 |
| 7 | 13.7 | <0.1 | <0.1 |
| 8 | 14.2 | 0.10 | <0.1 |
| 9 | 32.1 | 0.13 | <0.1 |
| 10 | 46.0 | <0.1 | <0.1 |
| 11 | 1.4 | <0.1 | <0.1 |
| 12 | 0.4 | <0.1 | <0.1 |
| 13 | < 0.1 | < 0.1 | < 0.1 |
| 14 | 28.0 | < 0.1 | < 0.1 |
| 15 | 79.2 | <0.1 | <0.1 |
| 16 | 2.1 | < 0.1 | < 0.1 |
| 17 | 0.2 | < 0.1 | < 0.1 |
| 18 | <0.1 | <0.1 | <0.1 |
| 19 | <0.1 | <0.1 | <0.1 |
| 20 | <0.1 | <0.1 | <0.1 |
| 21 | 17.8 | 267 | 22.7 |
| 22 | 20.1 | 140 | 59.7 |
| 23 | 4.01 | 9.3 | <0.1 |
| 24 | 41.2 | 9.3 | < 0.1 |
| 25 | 82.6 | 0.1 | <0.1 |
| 26 | 64.5 | 0.2 | <0.1 |
| 27 | 83.1 | 0.8 | 0.9 |
| 28 | 17.2 | 1.6 | <0.1 |
| 29 | 38.5 | 6.0 | <0.1 |
| 30 | 142 | 0.7 | 0.8 |
| 31 | 135 | 2.2 | 2.4 |
| 32 | 151 | 1.7 | 8.8 |
| 33 | 24.5 | <0.1 | 10.4 |
| 34 | 19.1 | 0.92 | 0.6 |
| 35 | 7.5 | <0.1 | 1.3 |
| 36 | 37.4 | 0.39 | 0.2 |
| 37 | 236 | 6.21 | 2.2 |
| 38 | 2.3 | - | - |
| 39 | 13.9 | 0.53 | <0.1 |
| 40 | 75.2 | <0.1 | <0.1 |
| 41 | 34.3 | <0.1 | <0.1 |
| 42 | 33.9 | 205.8 | 7.8 |
| 43 | 12.6 | 88.4 | 3.70 |
| 44 | 1.3 | <0.1 | <0.1 |
| 45 | 6.6 | < 0.1 | < 0.1 |
| 46 | 1.4 | < 0.1 | < 0.1 |
| 47 | 2.4 | < 0.1 | < 0.1 |
| 48 | 1.5 | < 0.1 | < 0.1 |
| 49 | 29.8 | <0.1 | 3.3 |
| 50 | 67.4 | 50.5 | 2.7 |
| 51 | 14.4 | 2.0 | 0.1 |
| 52 | 44.1 | 7.5 | 0.3 |
| 53 | 161 | 8.4 | 1.3 |
| 54 | 30.6 | 1.4 | 0.1 |
| 55 | 27.1 | 0.7 | 2.4 |
| 56 | 57.9 | 4.9 | 0.8 |
| 57 | 11.7 | <0.1 | 0.3 |
| 58 | 39.1 | 2.6 | 0.2 |
| 59 | 40.3 | <0.1 | 4.0 |
| 60 | 106.2 | <0.1 | 8.2 |
| 61 | 59.8 | <0.1 | 2.8 |
| 62 | 5.2 | <0.1 | <0.1 |
| 63 | 15.3 | <0.1 | <0.1 |
| 64 | 64.6 | 60.1 | 92.9 |
| 65 | 95.4 | 25.2 | 11.6 |
| 66 | 15.8 | 172 | 17.2 |
| 67 | 28.5 | 46.2 | 39.8 |
| 68 | 27.9 | 8.8 | 107 |
| 69 | 24.3 | 9.6 | 62.8 |
| 70 | 15.1 | 71.3 | 64.4 |
| 71 | 90.1 | 12.7 | 94.7 |
| 72 | 11.5 | 1.0 | 1.6 |
| 73 | 22.6 | 5.4 | 3.0 |
| 74 | 12.9 | 0.9 | 1.0 |
| 75 | 35.1 | 8.5 | 18.0 |
| 76 | 10.3 | 47.6 | 11.7 |
| 77 | 38.7 | 12.2 | 35.5 |
| 78 | 51.0 | 14.0 | 0.12 |
| 79 | 41.5 | 4.9 | 1.4 |
| 80 | 8.1 | 0.0 | 0.1 |
| 81 | 7.8 | 0.3 | <0.1 |
| 82 | 9.5 | 1.1 | <0.1 |
| 83 | 47.3 | 1.3 | 0.4 |
| 84 | 4.2 | <0.1 | <0.1 |
| 85 | 4.3 | <0.1 | 0.3 |
| 86 | 28.4 | 0.4 | 0.2 |
| 87 | 0.9 | <0.1 | <0.1 |
| 88 | 9.6 | 0.3 | <0.1 |
| 89 | 7.1 | 0.7 | <0.1 |
| 90 | 7.4 | <0.1 | <0.1 |
| 91 | 31.9 | 16.8 | 0.3 |
| 92 | 0.8 | <0.1 | 0.4 |
| 93 | 5.7 | 0.3 | 0.7 |
| 94 | 0.5 | <0.1 | <0.1 |
| 95 | 2.1 | 0.4 | <0.1 |
| 96 | 34.4 | 194.8 | 5.2 |
| 97 | 10.5 | 62.8 | 2.6 |
| 98 | 28.1 | 8.2 | 47.1 |
| 99 | 20.9 | 14.9 | 57.7 |
| 100 | 42.2 | 12.7 | 118.5 |
| 101 | 23.2 | 13.9 | 40.1 |
| 102 | 23.3 | 29.5 | 58.0 |

**[Table 3]**

| Relative Titer Ratio of Long-Acting Conjugates of Triple Agonists | | | |
|---|---|---|---|
| Long-Acting Conjugate | *in vitro* Activity Compared to Native Peptide (%) | | |
| | vs GLP-1 | vs Glucagon | vs GIP |
| Long-acting conjugate of SEQ ID NO: 21 | 0.1 | 1.6 | 0.2 |
| Long-acting conjugate of SEQ ID NO: 22 | 0.1 | 0.9 | 0.5 |
| Long-acting conjugate of SEQ ID NO: 42 | 3.1 | 23.1 | 1.2 |
| Long-acting conjugate of SEQ ID NO: 43 | 2.1 | 13.5 | 0.6 |
| Long-acting conjugate of SEQ ID NO: 50 | 15.4 | 6.9 | 0.7 |
| Long-acting conjugate of SEQ ID NO: 77 | 6.7 | 1.7 | 6.6 |
| Long-acting conjugate of SEQ ID NO: 96 | 0.3 | 4.0 | 0.3 |

The novel long-acting conjugates of the triple agonists prepared above had high activity on the glucagon receptor, increased targeting to liver cells, and also activated all of the glucagon receptor, GLP-1 receptor, and GIP receptor, and thus can be used as a therapeutic agent for the desired diseases in the liver.

### Experimental Example 2: Confirmation of Distribution of Long-Acting Conjugates of Triple Agonists in Tissues

The long-acting conjugate of SEQ ID NO: 42 was selected as a representative liver-targeted drug in three SD rats, and the distribution of the liver-targeted drug in tissues and organs was compared.

Specifically, the organs were extracted at 4, 48, and 168 hours after subcutaneously injecting 544 µg/kg of the long-acting conjugate of the triple agonist, and the concentration of each substance in tissues (serum, brain, pancreas, heart, kidney, stomach, small intestine, large intestine, lung, liver, spleen, adipose tissue, and muscle) was measured and compared by the ELISA method.

As a result, the long-acting conjugate of the triple agonist had the strongest tissue distribution detected at 48 hours after administration, and showed a particularly high distribution rate in the liver. Tissue distribution was the highest in the following order: liver, heart, lung, large intestine, spleen, pancreas, adipose tissue, small intestine, stomach, and muscle. The long-acting conjugate of the triple agonist was still detected at the highest rate in the liver even at 168 hours after administration, implying that the conjugate was present at a high rate in the liver until the 7th day after administration. Table 4 below summarizes the results of the tissue distribution ratio relative to serum of the long-acting conjugate of SEQ ID NO: 42 confirmed in the Examples above.

**[Table 4]**

| **Tissues** | 4 hours | | 48 hours | | 168 hours | |
|---|---|---|---|---|---|---|
| | Concentratio n (ng/ml, ng/g) | Distribution ratio T/S (%) | Concentratio n (ng/ml, ng/g) | Distribution ratio T/S (%) | Concentratio n (ng/ml, ng/g) | Distribution ratio T/S (%) |
| **Serum** | 167.0 ± 42.2 | - | 2281.7 ± 525.3 | - | 1113.3 ± 351.7 | - |
| **Liver** | 70.9 ±20.9 | 42.4 | 1143.4 ± 310.4 | 50.1 | 709.3 ± 42.7 | 63.7 |
| **Heart** | 30.6 ± 2.8 | 18.3 | 403.7 ± 80.5 | 17.7 | 108.1 ± 3.2 | 9.7 |
| **Lung** | <LLOQ | - | 311.6 ± 27.2 | 13.7 | 182.3 ± 12.7 | 16.4 |
| **Large intestine** | <LLOQ | - | 182.2 ± 23.0 | 8.0 | 52.2 ± 5.1 | 4.7 |
| **Spleen** | <LLOQ | - | 176.4 ± 35.0 | 7.7 | 130.3 ± 13.9 | 11.7 |
| **Pancrea s** | <LLOQ | - | 100.5 ± 101.5 | 4.4 | 39.8 ± 3.0 | 3.6 |
| **Adipose tissue** | <LLOQ | - | 101.2 ± 1.5 | 4.4 | 58.1 ± 6.0 | 5.2 |
| **Small intestine** | <LLOQ | - | 94.8 ±38.2 | 4.2 | <LLOQ | - |
| **Stomach** | <LLOQ | - | 67.7 ± 12.4 | 3.0 | 31.8 ± 3.3 | 2.9 |
| **Muscle** | <LLOQ | - | 68.4 ±34.2 | 3.0 | 41.5 ± 5.2 | 3.7 |
| **Kidney** | <LLOQ | - | <LLOQ | - | <LLOQ | - |
| **Brain** | <LLOQ | - | <LLOQ | - | <LLOQ | - |

| | | | | | | |
|---|---|---|---|---|---|---|
| * LLOQ (Lower Limit of Quantification) * T/S (tissue-to-serum ratio) * ng/ml indicates serum concentration, ng/g indicates tissue concentration | | | | | | |

The above results suggest that the long-acting conjugate of the triple agonist of the present invention has excellent tissue distribution in the liver compared to other tissues, and thus can be used as a therapeutic agent for the desired diseases. Therefore, the long-acting conjugate of the triple agonist may be used for a new purpose that can optimize drug treatment by inducing targeting to liver tissue so as to effectively deliver the required amount of drug. Additionally, as disclosed in International Publication No. WO 2020/263063, the triple agonist of the present invention or the long-acting conjugate thereof having excellent effects on liver diseases, e.g., metabolic liver disease, simple steatosis, non-alcoholic fatty liver, liver inflammation, non-alcoholic steatohepatitis, cholestatic liver disease, liver fibrosis, cirrhosis, liver failure and liver cancer, *etc.,* also exhibits excellent effect of targeting the long-acting conjugate of the triple agonist to the liver tissue and thereby has excellent preventive or therapeutic effects on the above liver diseases.

Further, the above results suggest that a high distribution of the long-acting conjugate of the triple agonist in the liver was observed even on the 7th day after administration, supporting the convenience of the conjugate as a preparation that can be administered about once a week.

From the foregoing, a skilled person in the art to which the present invention pertains will be able to understand that the present invention may be embodied in other specific forms without modifying the technical concepts or essential characteristics of the present invention. In this regard, the exemplary embodiments disclosed herein are only for illustrative purposes and should not be construed as limiting the scope of the present invention. On the contrary, the present invention is intended to cover not only the exemplary embodiments but also various alternatives, modifications, equivalents, and other embodiments that may be included within the spirit and scope of the present invention as defined by the appended claims.

## Claims

1. A pharmaceutical composition comprising a liver-targeted drug, which has a high distribution of the drug in the liver among the body organs of an administered subject,
wherein the liver-targeted drug is a peptide comprising the amino acid sequence represented by General Formula 1 below: 
Xaa1-Xaa2-Xaa3-Gly-Thr-Phe-Xaa7-Ser-Asp-Xaa10-Ser-Xaa12-Xaa13-Xaa14-
Xaa15-Xaa16-Xaa17-Xaa18-Xaa19-Xaa20-Xaa21-Phe-Xaa23-Xaa24-Trp-Leu-Xaa27-X
aa28-Xaa29-Xaa30-R1 (General Formula 1, SEQ ID NO: 103)
wherein in the General Formula 1 above,
Xaa1 is histidine (His, H), 4-imidazoacetyl (CA), or tyrosine (Tyr, Y);
Xaa2 is glycine (Gly, G), α-methyl-glutamic acid, or aminoisobutyric acid (Aib);
Xaa3 is glutamic acid (Glu, E) or glutamine (Gln, Q);
Xaa7 is threonine (Thr, T) or isoleucine (Ile, I);
Xaa10 is leucine (Leu, L), tyrosine (Tyr, Y), lysine (Lys, K), cysteine (Cys, C), or valine (Val, V);
Xaa12 is lysine (Lys, K), serine (Ser, S), or isoleucine (Ile, I);
Xaa13 is glutamine (Gln, Q), tyrosine (Tyr, Y), alanine (Ala, A), or cysteine (Cys, C);
Xaa14 is leucine (Leu, L), methionine (Met, M), or tyrosine (Tyr, Y);
Xaa15 is cysteine (Cys, C), aspartic acid (Asp, D), glutamic acid (Glu, E), or leucine (Leu, L);
Xaa16 is glycine (Gly, G), glutamic acid (Glu, E), or serine (Ser, S);
Xaa17 is glutamine (Gln, Q), arginine (Arg, R), isoleucine (Ile, I), glutamic acid (Glu, E), cysteine (Cys, C), or lysine (Lys, K);
Xaa18 is alanine (Ala, A), glutamine (Gln, Q), arginine (Arg, R), or histidine (His, H);
Xaa19 is alanine (Ala, A), glutamine (Gln, Q), cysteine (Cys, C), or valine (Val, V);
Xaa20 is lysine (Lys, K), glutamine (Gln, Q), or arginine (Arg, R);
Xaa21 is glutamic acid (Glu, E), glutamine (Gln, Q), leucine (Leu, L), cysteine (Cys, C), or aspartic acid (Asp, D);
Xaa23 is isoleucine (Ile, I) or valine (Val, V);
Xaa24 is alanine (Ala, A), glutamine (Gln, Q), cysteine (Cys, C), asparagine (Asn, N), aspartic acid (Asp, D), or glutamic acid (Glu, E);
Xaa27 is valine (Val, V), leucine (Leu, L), lysine (Lys, K) or methionine (Met, M);
Xaa28 is cysteine (Cys, C), lysine (Lys, K), alanine (Ala, A), asparagine (Asn, N), or aspartic acid (Asp, D);
Xaa29 is cysteine (Cys, C), glycine (Gly, G), glutamine (Gln, Q), threonine (Thr, T), glutamic acid (Glu, E), or histidine (His, H);
Xaa30 is cysteine (Cys, C), glycine (Gly, G), lysine (Lys, K), or histidine (His, H), or is absent; and
R1 is cysteine (Cys, C), GKKNDWKHNIT (SEQ ID NO: 106), m-SSGAPPPS-n (SEQ ID NO: 107), or m-SSGQPPPS-n (SEQ ID NO: 108), or is absent;
wherein,
m is Cys, Pro, or Gly-Pro;
n is Cys, Gly, Ser, or His-Gly, or is absent.

2. The pharmaceutical composition of claim 1, wherein the peptide is in the form of a long-acting conjugate, and the long-acting conjugate is represented by Formula 1 below:
[Formula 1] X-L-F
wherein,
X is a peptide comprising the amino acid sequence of General Formula 1 above;
L is a linker containing an ethylene glycol repeating unit;
F is an immunoglobulin Fc region; and
- represents a covalent bond between X and L, and between L and F.

3. The pharmaceutical composition of claim 1 or 2, wherein the body organ is the liver, heart, lung, large intestine, spleen, pancreas, adipose tissue, small intestine, stomach, muscle, kidney and brain, and the composition is the most highly distributed in the liver among each body organ.

4. The pharmaceutical composition of claim 1 or 2, for use in the prevention or treatment of diseases requiring drug action in the liver.

5. The pharmaceutical composition of claim 1 or 2, wherein the liver-targeted drug has a tissue-to-serum ratio (T/S ratio) in the liver after administration of at least one selected from the following:
(a) a T/S ratio of 25% to 50% at 2 to 5 hours after administration;
(b) a T/S ratio of 40% to 60% at 40 to 50 hours after administration; and
(c) a T/S ratio of 45% to 75% at 160 to 180 hours after administration.

6. The pharmaceutical composition of claim 5, wherein the liver-targeted drug has a T/S ratio in the liver after administration of at least one selected from the following:
(a) a T/S ratio of 35% to 45% at 4 hours after administration;
(b) a T/S ratio of 45% to 55% at 2 days after administration; and
(c) a T/S ratio of 55% to 70% at 7 days after administration.

7. The pharmaceutical composition of claim 1 or 2, wherein the liver-targeted drug has a distribution ratio in the liver relative to lung tissue after administration of 1:2 to 4.

8. The pharmaceutical composition of claim 7, wherein the liver-targeted drug has a distribution ratio in the liver relative to lung tissue after administration of 1:3 to 4.

9. The pharmaceutical composition of claim 8, wherein the liver-targeted drug has a distribution ratio in the liver relative to lung tissue after administration of 1: 3.5 to 4.

10. The pharmaceutical composition of claim 7, wherein the distribution ratio is the distribution ratio at 40 to 180 hours after administration.

11. The pharmaceutical composition of claim 9, wherein the distribution ratio is the distribution ratio at 2 to 7 days after administration.

12. The pharmaceutical composition of claim 1 or 2, wherein the liver-targeted drug has one of the following:
(a) a distribution ratio in the liver relative to the heart of 1:1.5 to 3.0 at 2 to 5 hours after administration;
(b) a distribution ratio in the liver relative to the heart of 1:2.0 to 3.0 at 40 to 50 hours after administration; and
(c) a distribution ratio in the liver relative to the heart at 160 to 180 hours after administration of 1: 5.5 to 7.0.

13. The pharmaceutical composition of claim 1 or 2, wherein the liver-targeted drug has therapeutic activity for liver diseases.

14. The pharmaceutical composition of claim 1 or 2, wherein
Xaa14 is leucine or methionine; and
Xaa15 is cysteine, aspartic acid, or leucine.

15. The pharmaceutical composition of claim 1 or 2,
wherein in the General Formula 1,
Xaa2 is glycine, α-methyl-glutamic acid, or Aib;
Xaa7 is threonine;
Xaa10 is tyrosine, cysteine, or valine;
Xaa12 is lysine or isoleucine;
Xaa13 is tyrosine, alanine, glutamine, or cysteine;
Xaa14 is leucine, tyrosine, or methionine;
Xaa15 is cysteine, leucine, glutamic acid, or aspartic acid;
Xaa17 is glutamine, arginine, isoleucine, cysteine, glutamic acid, or lysine;
Xaa18 is alanine, glutamine, arginine, or histidine;
Xaa19 is alanine, glutamine, valine, or cysteine;
Xaa20 is lysine, arginine, or glutamine;
Xaa21 is glutamic acid, glutamine, leucine, cysteine, or aspartic acid;
Xaa23 is isoleucine or valine;
Xaa24 is cysteine, alanine, glutamine, asparagine, glutamic acid, or aspartic acid; and
Xaa27 is leucine or lysine.

16. The pharmaceutical composition of claim 1 or 2, wherein the peptide comprises the amino acid sequence represented by General Formula 2 below: 
Xaa1-Xaa2-Gln-Gly-Thr-Phe-Thr-Ser-Asp-Xaa10-Ser-Lys-Xaa13-Xaa14-Xaa15-
Xaa16-Xaa17-Xaa18-Xaa19-Xaa20-Xaa21-Phe-Xaa23-Xaa24-Trp-Leu-Leu-Xaa28-Xaa
29-Xaa30-Xaa31-Ser-Ser-Gly-Gln-Pro-Pro-Pro-Ser-Xaa40 (General Formula 2, SEQ ID
NO: 104)
 wherein in the General Formula 2,
Xaa1 is 4-imidazoacetyl, histidine, or tyrosine;
Xaa2 is glycine, α-methyl-glutamic acid, or Aib;
Xaa10 is tyrosine or cysteine;
Xaa13 is alanine, glutamine, tyrosine, or cysteine;
Xaa14 is leucine, methionine, or tyrosine;
Xaa15 is aspartic acid, glutamic acid, or leucine;
Xaa16 is glycine, glutamic acid, or serine;
Xaa17 is glutamine, arginine, isoleucine, glutamic acid, cysteine, or lysine;
Xaa18 is alanine, glutamine, arginine, or histidine;
Xaa19 is alanine, glutamine, cysteine, or valine;
Xaa20 is lysine, glutamine, or arginine;
Xaa21 is cysteine, glutamic acid, glutamine, leucine, or aspartic acid;
Xaa23 is isoleucine or valine;
Xaa24 is cysteine, alanine, glutamine, asparagine, or glutamic acid;
Xaa28 is lysine, cysteine, asparagine, or aspartic acid;
Xaa29 is glycine, glutamine, cysteine, or histidine;
Xaa30 is cysteine, glycine, lysine, or histidine;
Xaa31 is proline or cysteine; and
Xaa40 is cysteine, or is absent.

17. The pharmaceutical composition of claim 1 or 2,
wherein in the General Formula 1,
Xaa2 is glycine, α-methyl-glutamic acid, or Aib;
Xaa7 is threonine;
Xaa10 is tyrosine, cysteine, or valine;
Xaa12 is lysine or isoleucine;
Xaa13 is tyrosine, alanine, or cysteine;
Xaa14 is leucine or methionine;
Xaa15 is cysteine or aspartic acid;
Xaa17 is glutamine, arginine, isoleucine, cysteine, or lysine;
Xaa18 is alanine, arginine, or histidine;
Xaa19 is alanine, glutamine, or cysteine;
Xaa20 is lysine or glutamine;
Xaa21 is glutamic acid, cysteine, or aspartic acid;
Xaa23 is valine;
Xaa24 is alanine, glutamine, cysteine, asparagine, or aspartic acid; and
Xaa27 is leucine or lysine.

18. The pharmaceutical composition of claim 16,
wherein in the General Formula 2,
Xaa13 is alanine, tyrosine, or cysteine;
Xaa15 is aspartic acid or glutamic acid;
Xaa17 is glutamine, arginine, cysteine, or lysine;
Xaa18 is alanine, arginine, or histidine;
Xaa21 is cysteine, glutamic acid, glutamine, or aspartic acid;
Xaa23 is isoleucine or valine;
Xaa24 is cysteine, glutamine, or asparagine;
Xaa28 is cysteine, asparagine, or aspartic acid;
Xaa29 is glutamine, cysteine, or histidine; and
Xaa30 is cysteine, lysine, or histidine.

19. The pharmaceutical composition of claim 1 or 2,
wherein in the General Formula 1,
Xaa2 is α-methyl-glutamic acid or Aib;
Xaa7 is threonine;
Xaa10 is tyrosine or cysteine;
Xaa12 is lysine or isoleucine;
Xaa13 is tyrosine, alanine, or cysteine;
Xaa14 is leucine or methionine;
Xaa15 is cysteine or aspartic acid;
Xaa16 is glutamic acid;
Xaa17 is arginine, isoleucine, cysteine, or lysine;
Xaa18 is alanine, arginine, or histidine;
Xaa19 is alanine, glutamine, or cysteine;
Xaa20 is lysine or glutamine;
Xaa21 is glutamic acid or aspartic acid;
Xaa23 is valine;
Xaa24 is glutamine, asparagine, or aspartic acid;
Xaa27 is leucine; and
Xaa28 is cysteine, alanine, asparagine, or aspartic acid.

20. The pharmaceutical composition of claim 1 or 2,
wherein in the General Formula 1,
Xaa1 is histidine or 4-imidazoacetyl;
Xaa2 is α-methyl-glutamic acid or Aib;
Xaa3 is glutamine;
Xaa7 is threonine;
Xaa10 is tyrosine;
Xaa12 is isoleucine,
Xaa13 is alanine or cysteine;
Xaa14 is methionine,
Xaa15 is aspartic acid;
Xaa16 is glutamic acid;
Xaa17 is isoleucine or lysine;
Xaa18 is alanine or histidine;
Xaa19 is glutamine or cysteine;
Xaa20 is lysine;
Xaa21 is aspartic acid;
Xaa23 is valine,
Xaa24 is asparagine;
Xaa27 is leucine;
Xaa28 is alanine or asparagine;
Xaa29 is glutamine or threonine; and
Xaa30 is cysteine or lysine, or is absent.

21. The pharmaceutical composition of claim 1 or 2, wherein the peptide is a peptide comprising the amino acid sequence of General Formula 3 below: Xaa1-Xaa2-Gln-Gly-Thr-Phe-Thr-Ser-Asp-Tyr-Ser-Lys-Xaa13-Leu-Asp-Glu-Xaa 
17-Xaa18-Xaa19-Lys-Xaa21-Phe-Val-Xaa24-Trp-Leu-Leu-Xaa28-Xaa29-Xaa30-Xaa31-
Ser-Ser-Gly-Gln-Pro-Pro-Pro-Ser-Xaa40 (General Formula 3, SEQ ID NO: 105)
 wherein in the General Formula 3 above,
Xaa1 is histidine or tyrosine;
Xaa2 is α-methyl-glutamic acid or Aib;
Xaa13 is alanine, tyrosine or cysteine;
Xaa17 is arginine, cysteine, or lysine;
Xaa18 is alanine or arginine;
Xaa19 is alanine or cysteine;
Xaa21 is glutamic acid or aspartic acid;
Xaa24 is glutamine or asparagine;
Xaa28 is cysteine or aspartic acid;
Xaa29 is cysteine, histidine, or glutamine;
Xaa30 is cysteine or histidine;
Xaa31 is proline or cysteine; and
Xaa40 is cysteine, or is absent.

22. The pharmaceutical composition of claim 1 or 2, wherein R1 is cysteine, GKKNDWKHNIT (SEQ ID NO: 106), CSSGQPPPS (SEQ ID NO: 109), GPSSGAPPPS (SEQ ID NO: 110), GPSSGAPPPSC (SEQ ID NO: 111), PSSGAPPPS (SEQ ID NO: 112), PSSGAPPPSG (SEQ ID NO: 113), PSSGAPPPSHG (SEQ ID NO: 114), PSSGAPPPSS (SEQ ID NO: 115), PSSGQPPPS (SEQ ID NO: 116), or PSSGQPPPSC (SEQ ID NO: 117), or is absent.

23. The pharmaceutical composition of claim 1 or 2, wherein the peptide comprises an amino acid sequence selected from the group consisting of SEQ ID NOS: 1 to 102.

24. The pharmaceutical composition of claim 1 or 2, wherein the Xaa16 amino acid and the Xaa20 amino acid of the General Formula 1 form a ring with each other.

25. The pharmaceutical composition of claim 1 or 2, wherein the peptide is amidated at its C-terminus or has a free carboxyl group (-COOH).

26. The pharmaceutical composition of claim 22, wherein the peptide is amidated at its C-terminus.

27. The pharmaceutical composition of claim 23, wherein the peptide is amidated at its C-terminus.

28. The pharmaceutical composition of claim 2, wherein the F is an IgG Fc region.

29. The pharmaceutical composition of claim 2, wherein the L is polyethylene glycol.

30. The pharmaceutical composition of claim 2, wherein the formula weight of the ethylene glycol repeating unit in L is in the range of 1 to 100 kDa.

31. The pharmaceutical composition of claim 4, wherein the disease requiring drug action in the liver is a liver disease.

32. The pharmaceutical composition of claim 30, wherein the liver disease is a metabolic liver disease.

33. The pharmaceutical composition of claim 31, wherein the liver disease is at least one disease selected from the group consisting of simple steatosis, non-alcoholic fatty liver, liver inflammation, non-alcoholic steatohepatitis, cholestatic liver disease, liver fibrosis, cirrhosis, liver failure, and liver cancer.

34. The pharmaceutical composition of claim 33, wherein the cholestatic liver disease is any one selected from the group consisting of primary biliary cirrhosis, primary sclerosing cholangitis, and a combination thereof.

35. A method for inducing targeting the liver-targeted drug to the liver by administering the pharmaceutical composition of any one of claims 1 to 34 to a subject in need thereof.

36. A method for inducing an increase in distribution of the liver-targeted drug in the liver tissue by administering the pharmaceutical composition of any one of claims 1 to 34 to a subject in need thereof.
